# EUROPEAN PATENT APPLICATION

(11) **EP 4 725 527 A1**
(43) Date of publication of application: **15.04.2026**
(21) Application number: 24851479.6
(22) Date of filing: 05.07.2024
(51) Int. Cl.: A61M 5/315, A61M 5/20

(54) **INJECTION DEVICE**

(30) Priority: 09.08.2023 JP 2023130064
(71) Applicant: Taisei Kako Co., Ltd., Kita-ku Osaka-shi Osaka 531-0072 (JP)
(72) Inventor: NAKAKUBO, Yutaro, Ibaraki-shi, Osaka 567-0054 (JP); SASAKI, Ayako, Ibaraki-shi, Osaka 567-0054 (JP); UMEZAKI, Masaya, Ibaraki-shi, Osaka 567-0054 (JP); OGAMI, Takashi, Ibaraki-shi, Osaka 567-0054 (JP)
(74) Representative: DTS Patent- und Rechtsanwälte PartmbB
(86) International application number: PCT/JP2024/024339
(87) International publication number: WO 2025/033049

(57) **Abstract**

Provided is an injection device including a barrel receiving part in which the barrel is received with an injection needle protruding from one side of a housing; an engagement releasing mechanism configured to release engagement between a plunger and an engaging member by rotating the plunger and the engaging member around a center axis with respect to each other in a state in which the plunger is positioned at a terminal end point due to an urging force of a plunger urging part; and a rotation restricting mechanism configured to restrict a rotation speed of relative rotation between the plunger and the engaging member.

## Description

### CROSS-REFERENCE TO RELATED APPLICATIONS

The present application claims priority of Japanese Patent Application No. 2023-130064, the content of which is incorporated by reference into the description of the present application.

### FIELD

The present invention relates to an injection device capable of pulling an injection needle out of a housing during use and returning the injection needle to the housing after use.

### BACKGROUND

As an example of the injection device described above, there is an injection device described in, for example, Patent Literature 1. This injection device includes a syringe, an engaging member, a plunger urging part and a barrel urging part, a housing for receiving them, and a trigger part.

The syringe includes a barrel filled with a drug solution and provided with an injection needle at a distal end, and a plunger configured to move from a proximal end side to a distal end side of the barrel and be inserted into the barrel. A spiral-shaped inclined part is provided at the proximal end of the barrel. The plunger includes a rod body extending in a direction of the center axis, and first and second branches protruding from an outer circumferential surface of the rod body. The second branch is disposed closer to the distal end side in the direction of the center axis than the first branch.

The engaging member includes an engagement body having a tubular shape into which the rod body can be inserted, and an urging engagement part having an outer flange shape radially outwardly projecting from an outer circumferential surface of the engagement body. The urging engagement part is provided with a release hole through which the first branch can be inserted in the direction of the center axis. The second branch and the urging engagement part are engaged with each other so as to allow the plunger to be engaged with the engaging member.

Further, the plunger urging part urges the engaging member to the distal end side in the direction of the center axis. The barrel urging part urges the barrel to the proximal end side in the direction of the center axis. Also, the urging force of the barrel urging part is set weaker than the urging force of the plunger urging part.

The housing includes a barrel receiving part and a plunger receiving part. The barrel receiving part receives the barrel and the barrel urging part in this order in an area from the proximal end side to the distal end side in the direction of the center axis. Additionally, the plunger urging part, the engaging member, and the plunger are received in this order in the plunger receiving part in an area from the proximal end side to the distal end side in the direction of the center axis. Also, the engaging member is received within the plunger receiving part while resisting the urging of the plunger urging part. Further, the trigger part engages with the engaging member received in the plunger receiving part to hold the urging of the plunger urging part so as not to be released. The trigger part is operated to release engagement with the engaging member to release the urging of the plunger urging part.

With this injection device, the trigger part is operated to release engagement with the engaging member to release the urging of the plunger urging part. Then, the engaging member and the syringe are pushed to the distal end side in the direction of the center axis by the urging of the plunger urging part. Thus, the barrel moves to the distal end side within the barrel receiving part while compressing the barrel urging part, and thereby the injection needle protrudes from the barrel receiving part. Thus, the injection needle can be pierced, for example, into the skin.

When the barrel moving to the distal end side abuts on a part inside the barrel receiving part, the movement of the barrel is restricted. In contrast to this, due to the urging of the plunger urging part, the engaging member and the plunger move further to the distal end side in the direction of the center axis. Then, when the plunger moves to the distal end side within the barrel, the drug solution is discharged from the injection needle. Thereby, the drug solution can be injected into, for example, the skin.

Here, in the injection device described in Patent Literature 1, when the plunger reaches just before the distal end of the barrel, the second branch abuts on the inclined part and slides thereon so that the plunger rotates with respect to the engaging member. Therefore, the first branch and the release hole are arranged to have their circumferential positions aligned with each other, and engagement between the plunger and the engaging member is released. Accordingly, the syringe is released from the urging by the plunger urging part and pushed to the proximal end side by the urging of the barrel urging part. Thus, the injection needle returns to the housing.

### CITATION LIST

### Patent Literature

Patent Literature 1: WO 2021/162086 A

### SUMMARY

### Technical Problem

There is a case in which, for example, it is desired to hold the injection needle in a state of being pierced into the skin for a while after injection is completed in order to deal with a problem such as leaking of the drug solution from the pierced site. However, the injection device described in Patent Literature 1 does not have a function for holding the injection needle in the state being pierced into the skin for a while after injection is completed.

Therefore, an object of the present invention is to provide an injection device capable of reliably completing injection by holding the injection needle in the state of protruding from the housing for a while after injection is completed.

### Solution to Problem

The present invention is an injection device including: a barrel having a distal end with an injection needle; a syringe including a plunger that is inserted into the barrel so as to be reciprocable between a proximal end side of the barrel as a starting point and a distal end side of the barrel with the injection needle as a terminal end point; a housing having a tubular shape for receiving the syringe, the housing including a barrel receiving part on one side in a center axis direction so as to receive the barrel in a movable manner between a position at which the injection needle is received in the housing, and a position at which the barrel which has moved a certain distance from the position with the injection needle received toward one side to have the injection needle protruding from the one side of the housing, and a plunger receiving part on another side of the barrel receiving part to receive the plunger; a barrel urging part arranged in the barrel receiving part and configured to urge the barrel from the one side to the other side of the housing; an engaging member arranged in the plunger receiving part and configured to engage with the plunger; a plunger urging part configured to engage with the engaging member and urge the plunger from the starting point toward the terminal end point; an engagement releasing mechanism configured to release engagement between the plunger and the engaging member by rotating the plunger and the engaging member around a center axis with respect to each other in a state in which the barrel is received in the barrel receiving part with the injection needle protruding from the one side of the housing, and the plunger is positioned at the terminal end point due to an urging force of the plunger urging part; and a rotation restricting mechanism configured to restrict a rotation speed of relative rotation between the plunger and the engaging member in the engagement releasing mechanism.

The present invention can be configured such that the engagement releasing mechanism is configured to release the engagement between the plunger and the engaging member by allowing the engaging member to rotate around the center axis with respect to the plunger, and the rotation restricting mechanism is configured to restrict the rotation speed of the engaging member.

The present invention can be configured such that the engaging member includes an engaging rotation part that is configured to engage with the plunger and rotate around the center axis with respect to the plunger to release engagement with the plunger; the engagement releasing mechanism is configured to release the engagement between the plunger and the engaging member by allowing the engaging rotation part to rotate with respect to the plunger to release the engagement between the plunger and the engaging member; and the rotating restricting mechanism includes an engaging non-rotation part that does not rotate around the center axis with respect to the engaging rotation part, and a rotation restricting part that is arranged between the engaging rotation part and the engaging non-rotation part and is configured to restrict the rotation speed of the engaging rotation part with respect to the engaging non-rotation part to restrict the rotation speed of the engaging rotation part with respect to the engaging non-rotation part.

The present invention can be configured such that the rotation restricting part is an elastic body arranged in an elastically deformed state between the engaging rotation part and the engaging non-rotation part.

The present invention can be configured such that the engagement releasing mechanism is configured to allow the plunger to rotate around the center axis with respect to the engaging member to release the engagement between the plunger and the engaging member; and the rotation restricting mechanism is configured to restrict the rotation speed of the plunger.

The present invention can be configured such that the plunger includes: a plunger rotation part that is configured to engage with the engaging member, and rotate around the center axis with respect to the engaging member to release the engagement; a plunger non-rotation part that does not rotate around the center axis with respect to the plunger rotation part; and a rotation restricting part that is arranged between the plunger rotation part and the plunger non-rotation part, and is configured to restrict the rotation speed of the plunger rotation part with respect to the plunger non-rotation part, in which the engagement releasing mechanism is configured to release the engagement between the engaging member and the plunger rotation part by allowing the plunger rotation part to rotate with respect to the engaging member to release the engagement between the plunger and the engaging member, and the rotation restricting mechanism includes the plunger non-rotation part and the rotation restricting part, and is configured to restrict the rotation speed of the plunger rotation part with respect to the plunger non-rotation part.

The present invention can be configured such that the rotation restricting part is an elastic body arranged in an elastically deformed state between the plunger rotation part and the plunger non-rotation part.

### BRIEF DESCRIPTION OF THE DRAWINGS

Fig. 1 is a cross sectional view of an injection device according to a first embodiment of the present invention, showing an initial state and showing a plunger in an external view.
Fig. 2 is a cross sectional view of a housing as viewed in a first radial direction according to the same embodiment.
Fig. 3 is an exploded perspective view of a syringe according to the same embodiment.
Fig. 4 is a view showing a plunger body part according to the same embodiment, and is a partial enlarged view of a plunger base part.
Fig. 5 is a cross sectional view taken along line V-V in Fig. 4.
Fig. 6 is a perspective view of an engaging member according to the same embodiment.
Fig. 7 is a cross sectional view as viewed in a second radial direction of the engaging member according to the same embodiment.
Fig. 8 is a cross sectional view taken along line VIII-VIII in Fig. 1.
Fig. 9 is a cross sectional view taken along line IX-IX in Fig. 1.
Fig. 10 is a detailed view showing a state in which a plunger-receiving engagement part and a body engagement part are engaged with each other in Fig. 1.
Fig. 11 is a cross sectional view of the injection device according to the same embodiment, showing a state in which an injection needle protrudes from the housing, and showing the plunger in an external view.
Fig. 12 is a cross sectional view of the injection device according to the same embodiment, showing a state in which injection of a drug solution has been completed, and showing the plunger and the engaging member in an external view.
Fig. 13 is a detailed view showing a state in which the engagement between the plunger-receiving engagement part and the body engagement part has been released in Fig. 12.
Fig. 14 is a cross sectional view of the injection device according to the same embodiment, showing a state in which the engaging member rotates around a center axis with respect to the plunger, and showing the plunger and the engaging member in an external view.
Fig. 15 is a detailed view showing a state in which the engagement body of Fig. 14 has rotated around the center axis.
Fig. 16 is a cross sectional view taken along line XVI-XVI in Fig. 14.
Fig. 17 is a cross sectional view of the injection device according to the same embodiment, showing a state in which the injection needle has returned to the inside of the housing, and showing the plunger and the engaging member in an external view.
Fig. 18 is a cross sectional view of the plunger receiving part according to a second embodiment as viewed in a second radial direction.
Fig. 19 is a front view of the plunger body part according to the same embodiment.
Fig. 20 is a cross sectional view taken along line XX-XX in Fig. 19.
Fig. 21 is a cross sectional view of a rod body, a plunger ring, and an elastic body of a plunger.
Fig. 22 is a perspective view of the engaging member according to the same embodiment.
Fig. 23 is a cross sectional view of the engaging member according to the same embodiment as viewed in a second radial direction.
Fig. 24 is a front view of the injection device according to the same embodiment, showing the housing in cross section, and showing an initial state.
Fig. 25 is a cross sectional view taken along line XXV-XXV in Fig. 24.
Fig. 26 is a front view of the injection device according to the same embodiment, showing the housing in cross section, and showing a state in which injection of the drug solution has been completed.
Fig. 27 is a cross sectional view taken along line XXVII-XXVII in Fig. 26.
Fig. 28 is a front view of the injection device according to the same embodiment, showing the housing in cross section, and showing a state in which the plunger has rotated around the center axis with respect to the engaging member.
Fig. 29 is a cross sectional view taken along line XXIX-XXIX in Fig. 28.
Fig. 30 is a front view of the injection device according to the same embodiment, showing the housing in cross section, and showing a state in which the injection needle has returned to the inside of the housing.

### DESCRIPTION OF EMBODIMENTS

Hereinafter, a description will be given on embodiments of an injection device according to the present invention. First, a configuration of an injection device 1 according to a first embodiment will be described.

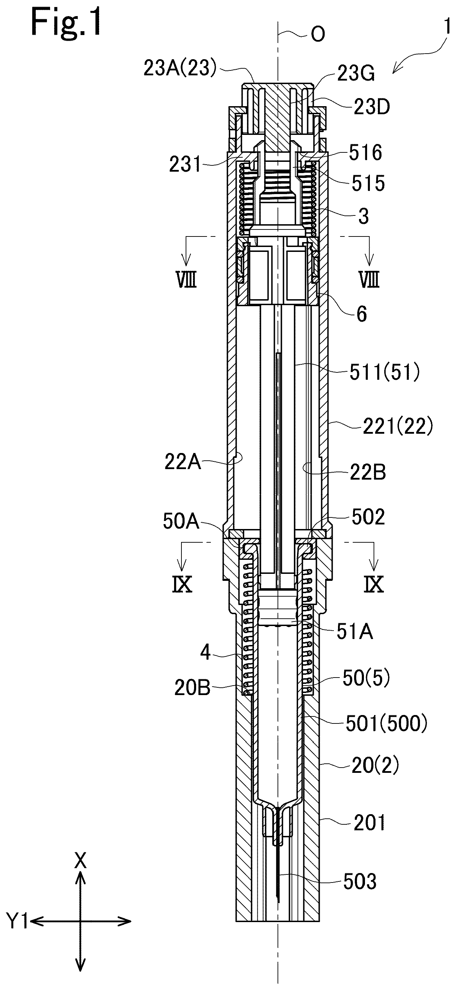

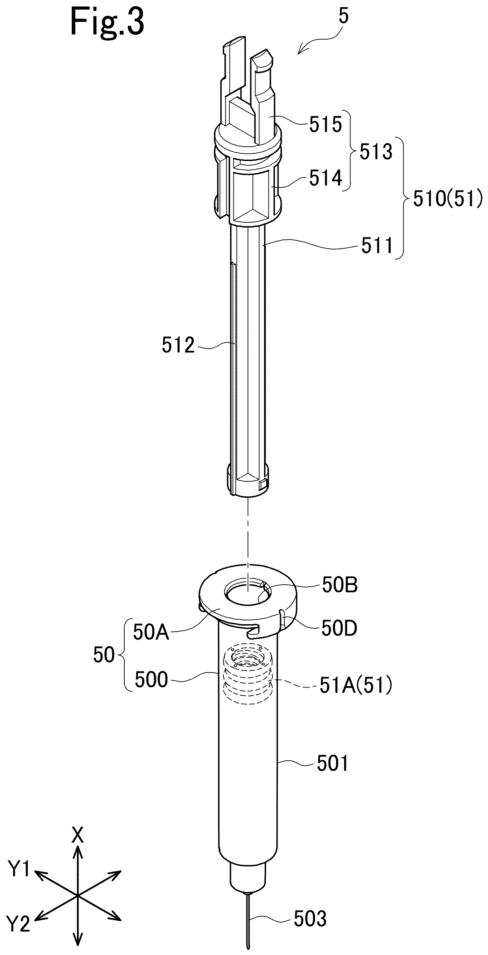

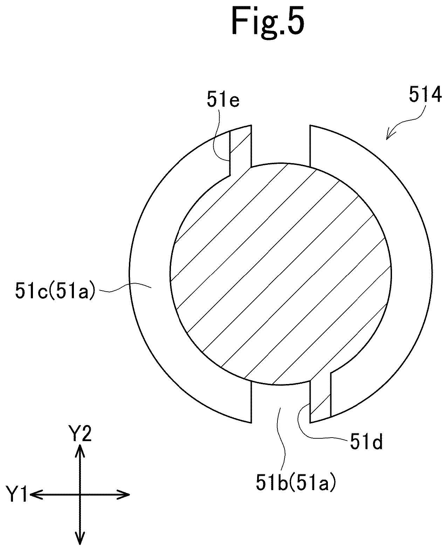

In the following description, the direction in which a center axis O of the injection device 1 extends will be referred to as a center axis direction X. The lower side of Fig. 1 will be referred to as one side in the center axis direction X, and the upper side of Fig. 1 will be referred to as another side in the center axis direction X. The direction orthogonal to the center axis direction X will be referred to as a radial direction. As shown in Figs. 3 and 5, the radial direction includes a first radial direction Y1 and a second radial direction Y2 which are orthogonal to each other. Further, the direction around the center axis O will be referred to as a circumferential direction.

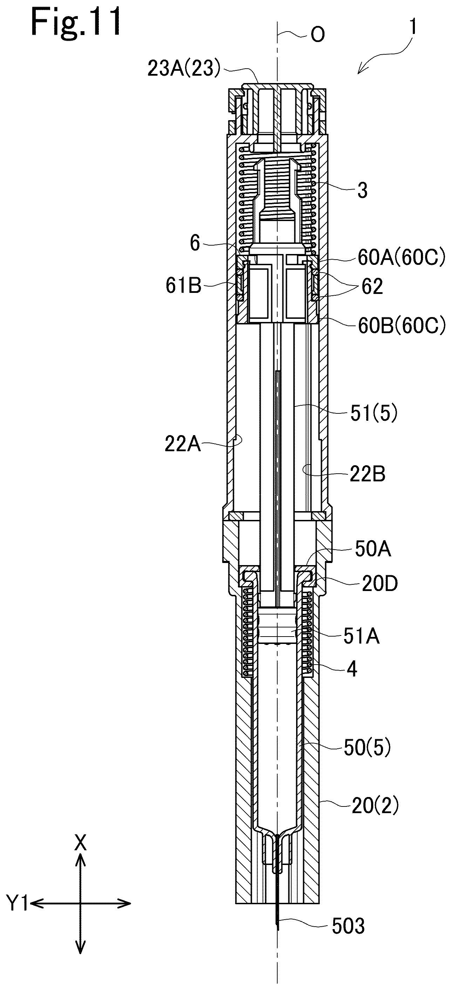

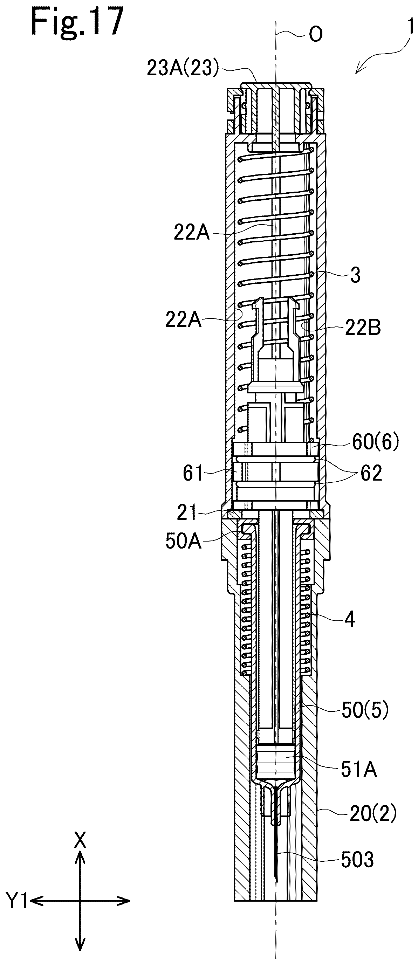

The injection device 1 is used, for example, when patients themselves inject a drug solution which is a liquid medication such as insulin or a rheumatism medication into their bodies. As shown in Figs. 11 and 17, this injection device 1 allows an injection needle 503 to protrude from a housing 2 during use, and return to the inside of the housing 2 after use. As shown in Fig. 1, the injection device 1 includes the housing 2, a plunger urging part 3, a barrel urging part 4, a syringe 5, and an engaging member 6.

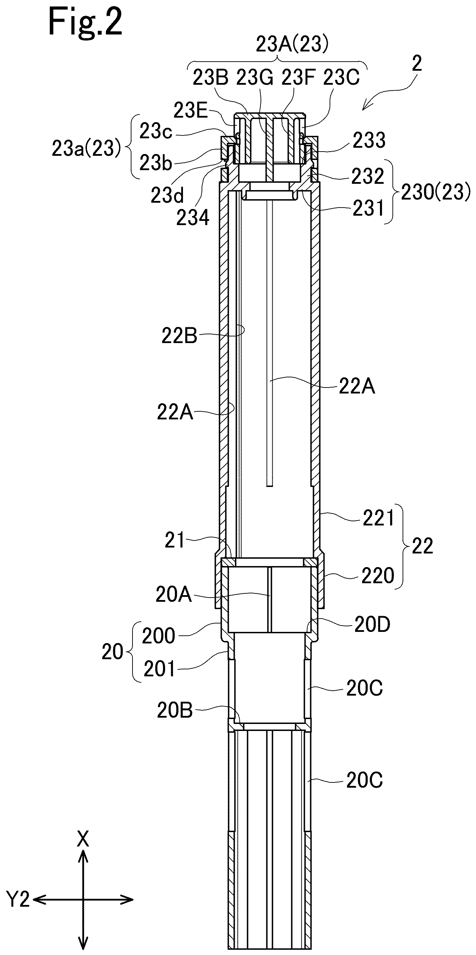

The housing 2 receives the plunger urging part 3, the barrel urging part 4, the syringe 5, and the engaging member 6. As shown in Fig. 2, the housing 2 is formed in a tubular shape (specifically, cylindrical tube shape) with one side opened and another side closed by a trigger part 23 described later in the center axis direction X. The housing 2 includes a barrel receiving part 20, a partition part 21, and a plunger receiving part 22.

As shown in Fig. 1, the barrel receiving part 20 receives the barrel 50. The barrel receiving part 20 has a tubular shape with openings on both sides in the center axis direction X. As shown in Fig. 2, the barrel receiving part 20 includes a barrel-other-side receiving part 200 having a tubular shape and arranged on the other side in the center axis direction X, and a barrel-one-side receiving part 201 having a tubular shape and arranged on one side in the center axis direction X.

An inner circumferential surface of the barrel-other-side receiving part 200 of this embodiment is provided with a barrel projection 20A protruding radially inward. This barrel projection 20A is formed over the entire area in the center axis direction X of the barrel-other-side receiving part 200. Also, the barrel projection 20A of this embodiment is formed along the center axis direction X. In this embodiment, a pair of the barrel projections 20A are arranged on the inner circumferential surface of the barrel-other-side receiving part 200 so as to face each other in the first radial direction Y1.

An inner circumferential surface of the barrel-one-side receiving part 201 is provided with an urging base part 20B having an internal flange shape and forming a through hole (not reference numeral allocated) in the center axis direction X. The urging base part 20B of this embodiment is arranged closer to the one side than the other side end in the center axis direction X of the barrel-one-side receiving part 201.

The barrel-one-side receiving part 201 of this embodiment is provided with a penetrating part 20C that penetrates radially. This penetrating part 20C is a through hole that penetrates the barrel-one-side receiving part 201 in the second radial direction Y2. The through hole 20C of this embodiment is configured in a rectangular shape along the circumferential surface of the barrel-one-side receiving part 201. In this embodiment, the through holes 20C are respectively provided on one side and the other side in the center axis direction X of the urging base part 20B. A pair of the through holes 20C are provided in the barrel-one-side receiving part 201 so as to face each other in the second radial direction Y2.

The barrel-one-side receiving part 201 is connected to one side in the center axis direction X of the barrel-other-side receiving part 200. Here, an inner diameter of the barrel-one-side receiving part 201 is smaller than the inner diameter of the barrel-other-side receiving part 200. Therefore, a barrel step part 20D, which is a step part continuous with the barrel-other-side receiving part 200, is formed at the other side end in the center axis direction X of the barrel-one-side receiving part 201. The barrel step part 20D is a flat surface that extends along the radial direction and in the circumferential direction.

The partition part 21 is composed of an annular member forming a through hole penetrating in the center axis direction X. This partition part 21 is a flat surface that has one side end and another side end in the center axis direction X both extending in the radial direction and in the circumferential direction. An inner diameter of the partition part 21 is smaller than the inner diameter of the barrel-other-side receiving part 200. On the other hand, an outer diameter of the partition part 21 is substantially the same as the outer diameter of the barrel-other-side receiving part 200. The partition part 21 is arranged to have one side end in the center axis direction X abutting on the other side end in the center axis direction X of the barrel receiving part 20.

The plunger receiving part 22 has a tubular shape extending in the center axis direction X. As shown in Fig. 2, the plunger receiving part 22 includes a plunger-one-side receiving part 220 having a tubular shape and arranged on one side in the center axis direction X, and a plunger-other-side receiving part 221 having a tubular shape and arranged on the other side in the center axis direction X.

The plunger-one-side receiving part 220 has a tubular shape with an inner diameter larger than the outer diameters of the barrel-other-side receiving part 200 and the partition part 21. Therefore, as shown in Fig. 2, the plunger-one-side receiving part 220 is fitted onto the barrel-other-side receiving part 200 and the partition part 21 from the other side in the center axis direction X, so that the plunger receiving part 22 is connected to the barrel receiving part 20 and the partition part 21. Thus, the housing 2 is configured.

A plunger receiving part (no reference numeral allocated) is provided on the inner circumferential surface of the plunger-other-side receiving part 221. The plunger-receiving engagement part of this embodiment includes a plunger-receiving projection 22A that protrudes radially inward from the plunger-other-side receiving part 221, and a plunger receiving recess 22B recessed into the plunger-other-side receiving part 221.

The plunger-receiving projection 22A is continuously formed from the other side end to the one side in the center axis direction X of the plunger-other-side receiving part 221. One side end in the center axis direction X of the plunger-receiving projection 22A is arranged on the other side in the center axis direction X of the one end of the plunger-other-side receiving part 221. Therefore, the plunger-receiving projection 22A is not formed over the entire area in the center axis direction X of the plunger-other-side receiving part 221, and is shorter than the plunger-other-side receiving part 221 in the center axis direction X. Further, the plunger-receiving projection 22A is formed to extend along the center axis direction X. In this embodiment, two pairs of the plunger-receiving projections 22A are provided on the inner circumferential surface of the plunger-other-side receiving part 221 such that those of one pair face each other in the first radial direction Y1 and those of another pair face each other in the second radial direction Y2. In contrast to this, the plunger receiving recess 22B is formed over the entire area in the center axis direction X of the plunger-other-side receiving part 221. This plunger receiving recess 22B is formed to extend along the center axis direction X. Also, the plunger receiving recess 22B is arranged between the plunger-receiving projections 22A which are adjacent to each other in the circumferential direction.

The plunger-other-side receiving part 221 is connected to the other side in the center axis direction X of the plunger-one-side receiving part 220, thereby forming the plunger receiving part 22. Here, an inner diameter of the plunger-other-side receiving part 221 is smaller than the inner diameter of the plunger-one-side receiving part 220. Therefore, a plunger step part (no reference numeral allocated), which is a step part continuous with the plunger-one-side receiving part 220, is formed at one side end in the center axis direction X of the plunger-other-side receiving part 221. The plunger step part is a flat surface that extends along the radial direction and in the circumferential direction.

Here, an inner diameter of the plunger-other-side receiving part 221 is smaller than the outer diameter of the partition part 21. Therefore, as shown in Fig. 2, the plunger step part abuts on the other side end in the center axis direction X of the partition part 21 in the housing 2. In this embodiment, the inner diameter of the plunger-other-side receiving part 221 is larger than the inner diameter of the partition part 21.

As shown in Figs. 1 and 2, the trigger part 23 is arranged on the other side in the center axis direction X of the plunger-other-side receiving part 221. The trigger part 23 includes a trigger mounting part 230, a trigger body part 23A, and a trigger lock operation part 23a.

As shown in Fig. 2, the trigger mounting part 230 includes a trigger mounting base part 231 protruding radially inward from the plunger-other-side receiving part 221, and a trigger tubular part 232 having a cylindrical tube shape and protruding from the trigger mounting base part 231 to the other side in the center axis direction X.

The trigger mounting base part 231 has an internal flange shape that forms an insertion hole in the center axis direction X. The trigger mounting base part 231 is arranged at the other side end in the center axis direction X of the plunger-other-side receiving part 221. An insertion hole (not shown) is provided at one side end in the center axis direction of the trigger mounting base part 231.

The trigger tubular part 232 is arranged to surround the insertion hole. A lock step part 233 is formed in the inner circumferential surface of the trigger tubular part 232. The lock step part 233 extends from the trigger mounting base part 231 in the center axis direction X over the middle of the trigger tubular part 232. A pair of the lock step parts 233 are provided substantially 180 degrees apart from each other in the circumferential direction. Also, a plurality of projections 234 protruding radially outward are formed on the outer circumferential surface of the trigger tubular part 232.

The trigger body part 23A includes a top plate part 23B that has a disc shape and is configured to be pushed down by the user's operation, an outer skirt part 23C that is suspended from an outer peripheral edge of the top plate part 23B to one side in the center axis direction X, an inner skirt part 23F that is suspended from the top plate part 23B on the radially inside of the outer skirt part 23C to one side in the center axis direction X, and a flat plate part 23G.

The outer skirt part 23C is formed in a cylindrical tube shape. As shown in Fig.1, a release groove 23D is formed in the outer skirt part 23C by cutting out a circumferential part of the outer skirt part 23C extending from one side end in the center axis direction X to the other side in the center axis direction X. The release groove 23D is configured to allow the lock step part 233 to be inserted thereinto. A pair of the release grooves 23D are formed in the outer skirt part 23C to face each other in the radial direction in this embodiment. As shown in Fig. 2, a recessed interlocking groove 23E is provided in the outer circumferential surface of the outer skirt part 23C. This interlocking groove 23E is provided to extend from the other side end to the one side in the center axis direction X of the outer skirt part 23C. A plurality of the interlocking grooves 23E are provided in the outer circumferential surface of the outer skirt part 23C.

An outer diameter of the outer skirt part 23C is smaller than the inner diameter of the trigger tubular part 232. Therefore, the trigger body part 23A is configured to be inserted into the trigger tubular part 232. The length of the outer skirt part 23C in the center axis direction X is the same as the length of the trigger tubular part 232 in the center axis direction X. In Fig. 2, with one side end in the center axis direction X of the outer skirt part 23C abutting on the lock step part 233, the trigger body part 23A is inserted into the trigger tubular part 232. Further, the outer skirt part 23C is configured to rotate around the center axis O while being inserted into the trigger tubular part 232. Therefore, the outer skirt part 23C inserted into the trigger tubular part 232 is rotated around the center axis O to have the circumferential positions of the lock step parts 233 and the release grooves 23D aligned with each other, so that the lock step parts 233 can be inserted into the release grooves 23D. Thus, when the circumferential positions of the release grooves 23D and the lock step parts 233 are aligned with each other, the trigger body 23A can be pushed into one side in the center axis direction X.

The inner skirt part 23F has a tubular shape. An outer diameter of the inner skirt part 23F is smaller than the inner diameter of the outer skirt part 23C. Therefore, the inner skirt part 23F is arranged inside of the outer skirt part 23C. The inner skirt part 23F has substantially the same length as the outer skirt part 23C in the center axis direction X.

The flat plate part 23G is a plate shaped member having a length dimension in the center axis direction X, and a width dimension and a thickness dimension in the radial direction. In Figs. 1 and 2, the flat plate part 23G has a width dimension in the first radial direction Y1 and a thickness dimension in the second radial direction Y2. The flat plate part 23G is suspended from the top plate part 23B on the radially inside of the inner skirt part 23F. The flat plate part 23G is longer in the center axis direction X than the inner skirt part 23F and the outer skirt part 23C. This flat plate part 23G is configured to be inserted into the insertion hole of the trigger mounting base part 231.

As shown in Fig. 2, the trigger lock operation part 23a includes a tubular part 23b having a cylindrical tube shape, and a protrusion 23c protruding radially inward from the other side end in the center axis direction X of the tubular part 23b. A rotation groove 23d extending in the circumferential direction is formed in the tubular part 23b by cutting out a part of a circumferential surface of the tubular part 23b. This rotation groove 23d is configured to allow the projections 234 to be inserted thereinto.

An inner diameter of the tubular part 23b is larger than the outer diameter of the trigger tubular part 232. Therefore, in Fig. 2, with the projections 234 inserted into the rotation groove 23d, the tubular part 23b is fitted onto the trigger tubular part 232. The protrusion 23c is configured to be inserted into the interlocking groove 23E. The protrusion 23c is configured to be inserted into the interlocking grooves 23E to prevent the trigger body part 23A from falling out of the trigger tubular part 232. A plurality of the protrusions 23c are provided in the circumferential direction in this embodiment. Here, the trigger lock operation part 23a is configured to rotate around the center axis O. Therefore, the trigger lock operation part 23a rotates around the center axis O so that the trigger body part 23A can be rotated around the center axis O.

The plunger urging part 3 is configured to urge the syringe 5 and the engaging member 6 from the other side to the one side in the center axis direction X. Also, the plunger urging part 3 of this embodiment is configured to urge the engaging member 6 to rotate it around the center axis O. The plunger urging part 3 of this embodiment is a compression coil torsion spring wound around the center axis O. An outer diameter of the plunger urging part 3 is smaller than the inner diameter of the plunger receiving part 22. Therefore, the plunger urging part 3 is configured to be received within the plunger receiving part 22. As shown in Fig. 1, the plunger urging part 3 is received within the plunger-one-side receiving part 220 with the other side end in the center axis direction X inserted into the insertion hole provided in the trigger mounting base part 231 from the one side in the center axis direction X.

The barrel urging part 4 is configured to urge the barrel 50 from one side to the other side in the center axis direction X. As shown in Fig. 1, the barrel urging part 4 of this embodiment is a compression coil spring wound around the center axis O. The barrel urging part 4 is configured to be received in the barrel receiving part 20. Specifically, the barrel urging part 4 has a larger diameter than the through hole formed by the urging base part 20B and a smaller diameter than the inner diameter of the barrel-one-side receiving part 201. Therefore, as shown in Fig. 1, the barrel urging part 4 is received in the barrel receiving part 20 while abutting on the urging base part 20B from the other side in the center axis direction X. Thus, the barrel urging part 4 is stopped from moving to the one side in the center axis direction X by the urging base part 20B. The urging force of the barrel urging part 4 is set to be weaker than the urging force of the plunger urging part 3.

As shown in Fig. 3, the syringe 5 includes a barrel 50 having a distal end with an injection needle 503, and a plunger 51. The barrel 50 includes a barrel body part 500 filled with a drug solution inside, and a cover part 50A attached to the barrel body part 500.

The barrel body part 500 is formed of a transparent resin, glass, or the like. The barrel body part 500 includes a barrel tubular part 501 filled with the drug solution and an outer flange 502 (see Fig. 1) protruding radially outward from the other side end in the center axis direction X of the barrel tubular part 501.

The barrel tubular part 501 has a cylindrical tube shape. As shown in Fig. 3, the injection needle 503 protrudes from one side end in the center axis direction X of the barrel tubular part 501. Therefore, the injection needle 503 is provided at one side end in the center axis direction X, which is the distal end of the barrel 50. In this embodiment, an outer diameter of the barrel tubular part 501 is smaller than the inner diameter of the barrel urging part 4, which is a compression coil spring. Therefore, as shown in Fig. 1, the barrel tubular part 501 is inserted into the barrel urging part 4. Also, the outer diameter of the barrel tubular part 501 is smaller than the through hole formed by the urging base part 20B. Therefore, the barrel body part 500 is arranged within the barrel receiving part 20 with the barrel tubular part 501 inserted into the through hole.

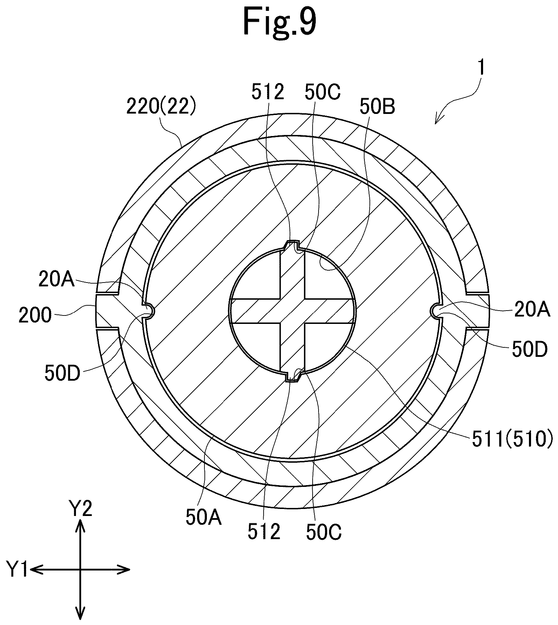

As shown in Fig. 3, the cover part 50A has a tubular shape with a part of the outer circumferential surface cut and a bottom surface formed in a substantially C-shape. This cover part 50A is a flat surface having one side end and the other side end in the center axis direction X extending along the radial direction and in the circumferential direction. A circular through hole 50B that penetrates in the center axis direction X is formed in the cover part 50A. As shown in Fig. 9, an axial movement groove 50C is formed in this through hole 50B by cutting out a part of the inner circumferential surface of the cover part 50A. In this embodiment, two axial movement grooves 50C are formed in the second radial direction Y2. Also, as shown in Figs. 3 and 9, a cover recess 50D recessed radially inward is provided in the outer circumferential surface of the cover part 50A. The cover recess 50D is provided along the center axis direction X. The cover recess 50D is formed from one side end to the other side end in the center axis direction X of the cover part 50A. In this embodiment, the cover part 50A is configured such that a pair of the cover recesses 50D face each other in the first radial direction Y1.

The cover part 50A is configured to be attached to the barrel body part 500. The cover part 50A of this embodiment is attached to the barrel body part 500 by sliding the outer flange 502 in the radial direction from the cut part of the outer circumferential surface into the cover part 50A. In the state in which the cover part 50A is attached to the barrel body part 500, the through hole 50B of the cover part 50A communicates with the inside of the barrel tubular part 501.

An outer diameter of the cover part 50A is substantially the same as the inner diameter of the barrel-other-side receiving part 200. Therefore, the cover part 50A is configured to be received in the barrel-other-side receiving part 200. Here, as shown in Fig. 9, the cover part 50A is received in the barrel-other-side receiving part 200, in the state in which the cover recesses 50D of the cover part 50A engage with the barrel projections 20A of the barrel-other-side receiving part 200.

As shown in Fig. 1, the length in the center axis direction X of the cover part 50A is shorter than the length in the center axis direction X of the barrel-other-side receiving part 200. Therefore, the cover part 50A can move in the center axis direction X within the barrel-other-side receiving part 200.

Here, the outer diameter of the cover part 50A is larger than the outer diameter of the barrel urging part 4, which is a compression coil spring. Therefore, in the state in which the barrel 50 is arranged within the barrel receiving part 20, the barrel urging part 4 abuts on one side end in the center axis direction X of the cover part 50A. Thus, the cover part 50A stops the urging of the barrel urging part 4 from the other side in the center axis direction X. Therefore, as shown in Figs. 11 and 17, the cover part 50A moves to the one side in the center axis direction X by resisting the urging force of the barrel urging part 4, and moves to the other side in the center axis direction X by the urging force of the barrel urging part 4.

The outer diameter of the cover part 50A is larger than the inner diameter of the barrel-one-side receiving part 201 and the inner diameter of the partition part 21. Therefore, the cover part 50A is configured to abut on the barrel step part 20D when moving to the one side in the center axis direction X within the barrel-other-side receiving part 200, and abuts on one side end in the center axis direction X of the partition part 21 when moving to the other side in the center axis direction X.

The length in the center axis direction X of the barrel 50 is shorter than the length in the center axis direction X of the barrel receiving part 20. Therefore, in the state shown in Fig. 1, the barrel 50 (specifically, the cover part 50A) is urged to the other side in the center axis direction X by the urging force of the barrel urging part 4, and the injection needle 503 is received within the housing 2. This state is referred to as a first state. On the other hand, in the state shown in Fig. 11, the barrel 50 (specifically, the cover part 50A) resists the urging force of the barrel urging part 4, and thereby the injection needle 503 protrudes from the one side in the center axis direction X of the housing 2. This state is referred to as a second state. That is, the barrel 50 is movable between the first state and the second state.

The plunger 51 is inserted into the barrel 50. As shown in Fig. 3, the plunger 51 includes a piston 51A, and a plunger body 510 configured to be engaged with the piston 51A.

The piston 51A prevents the drug solution in the barrel 50 from leaking out of the barrel 50 and pushes the drug solution out of the barrel 50 from the injection needle 503. The piston 51A is arranged within the barrel tubular part 501 and tightly contacts the entire circumference of the inner circumferential surface of the barrel tubular part 501.

The plunger body 510 includes a rod 511 and a plunger base part 513 arranged closer to the other side in the center axis direction X than the rod 511.

The rod 511 is a member extending along the center axis direction X. This rod 511 has a diameter smaller than the inner diameter of the barrel tubular part 501 and the through hole 50B of the cover part 50A. Therefore, the rod 511 is configured to be inserted into the barrel 50. Thus, as shown in Fig. 1, the plunger 51 is inserted into the barrel 50 in a state of being able to reciprocate between one side and the other side in the center axis direction X of the barrel 50.

An axial movement protrusion 512 protruding outward is formed on the outer circumferential surface of the rod 511. The axial movement protrusion 512 extends from one side end to the other side in the center axis direction X of the rod 511. The shaft movement protrusion 512 is configured to be fitted into the axial movement groove 50C. In this embodiment, a pair of the axial movement protrusions 512 are formed in the second radial direction Y2.

As shown in Fig. 9, the rod 511 is inserted into the through hole 50B with the shaft movement protrusions 512 respectively fitted into the axial movement grooves 50C, so the plunger body 510 is inserted into the barrel 50 in a state of being prevented from rotating around the center axis O. As shown in Fig. 1, one side end in the center axis direction X of the rod 511 abuts on the other side end in the center axis direction X of the piston 51A.

As shown in Fig. 3, the plunger base part 513 is arranged closer to the other side in the center axis direction X than the rod 511. The plunger base part 513 in this embodiment has a larger diameter than the rod 511. The plunger base part 513 includes a plunger engagement part 514 and a trigger engagement part 515.

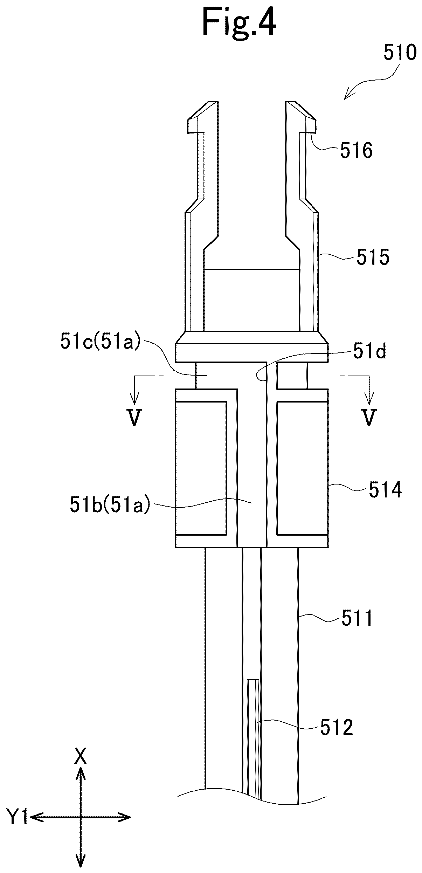

As shown in Fig. 4, the plunger engagement part 514 is connected to the other side end in the center axis direction X of the rod 511. The plunger engagement part 514 in this embodiment is a member with a larger diameter than the rod 511.

A groove 51a radially inwardly recessed is formed in the outer circumferential surface of the plunger engagement part 514. As shown in Figs. 4 and 5, the groove 51a of this embodiment includes an axial groove 51b extending in the center axis direction X and a circumferential groove 51c extending in the circumferential direction. The axial groove 51b is provided in the plunger engagement part 514 so as to extend from one side end to the other side in the center axis direction X of the plunger engagement part 514. This axial groove 51b extends along the center axis direction X. As shown in Figs. 4 and 5, the circumferential groove 51c is provided in the plunger engagement part 514 so as to extend along the circumferential direction. The circumferential groove 51c includes a circumferential one side end 51d, which is one side end in the circumferential direction, and a circumferential other side end 51e, which is another side end in the circumferential direction. The circumferential groove 51c is arranged closer to the other side in the center axis direction X than the axial groove 51b, and is continuous with the axial groove 51b in the center axis direction X. Specifically, the circumferential groove 51c has the circumferential one side end 51d continuous with the axial groove 51b.

As shown in Figs. 3 and 4, the trigger engagement part 515 is a member with the other side in the center axis direction X bifurcated. The trigger engagement part 515 is configured to bend so as to allow the bifurcated portions to come close to each other. The bent trigger engagement part 515 is configured to return to its original state. Thus, the trigger engagement part 515 is configured to be elastically deformed. A rib 516 protruding radially outward is formed at each of the other side ends in the center axis direction X of the trigger engagement part 515. This rib 516 is formed in a tapered shape with its amount of radially outward protrusion increasing as it advances from the other side to the one side in the center axis direction X. One side end of the rib 516 in the center axis direction X is a flat surface along the radial direction.

The trigger engagement part 515 is configured to be inserted through the insertion hole formed by the trigger mounting base part 231. Specifically, the rib 516 abuts on the inner circumferential surface of the trigger mounting base part 231 and bends so as to allow the bifurcated portions of the trigger engagement part 515 to come close to each other, and thereby the trigger engagement part 515 is inserted through the through hole formed by the trigger mounting base part 231, as shown in Fig. 1. Then, the trigger engagement part 515 returns to its original state due to its elastic force, and one side end in the center axis direction X of the rib 516 abuts on the trigger mounting base part 231 from the other side in the center axis direction X.

The distance between the bifurcated portions of the trigger engagement part 515 is longer than the width of the flat plate part 23G. Therefore, it is configured such that the flat plate part 23G can be inserted between the bifurcated portions of the trigger engagement part 515. In Fig. 1, the flat plate part 23G is arranged between the bifurcated portions of the trigger engagement part 515.

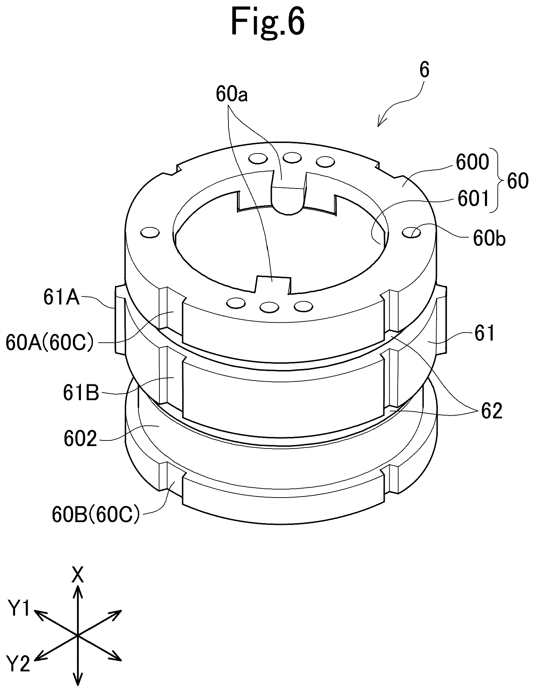

As shown in Fig. 6, the engaging member 6 is formed in an annular shape. The engaging member 6 includes an engagement body 60 having a tubular shape, an engaging ring 61, and an elastic body 62. The engagement body 60 of this embodiment includes an engaging annular part 600 and an engaging tubular part 601.

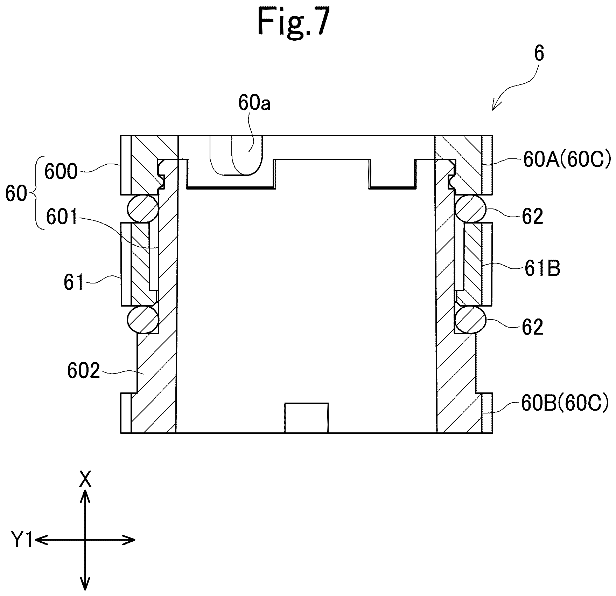

The engaging annular part 600 is a member having an annular shape. As shown in Fig. 7, in the engaging annular part 600 of this embodiment, one side end and the other side end in the center axis direction X are flat surfaces. An inner diameter of this engaging annular part 600 is larger than the plunger body 510. On the other hand, an outer diameter of the engaging annular part 600 is smaller than the inner diameter of the plunger-other-side receiving part 221.

A radially inward projection 60a radially inwardly protruding is formed in the engaging annular part 600. This radially inward projection 60a is configured to be inserted into the groove 51a of the plunger engagement part 514. As shown in Fig. 6, the engaging annular part 600 of this embodiment includes a plurality of (specifically, two) radially inward projections 60a arranged in the circumferential direction. A fixing part for fixing one side end in the center axis direction X of the plunger support part 3 is provided at the other side end in the center axis direction X of the engaging annular part 600. The fixing part of this embodiment is an insertion hole 60b into which one side end in the center axis direction X of the plunger urging part 3 can be inserted. Also, an annular recess 60A capable of fitting to the plunger-receiving projection 22A in a recess/projection way is formed in the outer circumferential surface of the engagement annular part 600. Two pairs of the annular recesses 60A are provided such that those of one pair are arranged in the first radial direction Y1 and those of another pair are arranged in the second radial direction Y2.

The engaging tubular part 601 has a tubular shape. As shown in Fig. 7, the engaging tubular part 601 is brought into connection with the engaging annular part 600 from one side in the center axis direction X. The inner diameter of the engaging tubular part 601 is substantially the same as the inner diameter of the engaging annular part 600. Therefore, the plunger body 510 can be inserted into the engagement body 60. An outer diameter of the engaging tubular part 601 is smaller than the outer diameter of the engaging annular part 600.

The outer circumferential surface of the engaging tubular part 601 is provided with a flange part 602 protruding radially outward. The flange part 602 in this embodiment is arranged on one side in the center axis direction X of the engaging tubular part 601. This flange part 602 is formed in a step shape to have one side in the center axis direction X positioned radially more outward than the other side. Therefore, the flange part 602 of this embodiment has a largest outer diameter at one side end in the center axis direction X. Also, the other side end and the one side end in the center axis direction X of the flange part 602 are flat surfaces that extend in the radial direction and in the circumferential direction. Further, the outer diameter of the flange part 602 at the one side end in the center axis direction X is substantially the same as the outer diameter of the engaging annular part 600. Therefore, the engagement body 60 can be received in the plunger-other-side receiving part 221.

The outer circumferential surface of the flange part 602 is provided with a flange recess 60B that can be fitted to the plunger-receiving projection 22A in a recess/projection way. This flange recess 60B is arranged at the one side end in the center axis direction X with the largest outer diameter of the flange part 602. The flange recess 60B is arranged at substantially the same position as that of the annular recess 60A in the circumferential direction. Therefore, two pairs of the flange recesses 60B are provided such that those of one pair are arranged in the first radial direction Y1 and those of another pair are arranged in the second radial direction Y2. In this embodiment, the body engagement part 60C capable of engaging with the plunger-receiving engagement part is composed of the flange recess 60B and the annular recess 60A.

The engaging ring 61 is a member having an annular shape. One side end and the other side end in the center axis direction X of the engaging ring 61 are flat surfaces. An inner diameter of the engaging ring 61 is larger than the outer diameter of the engaging tubular part 601. Therefore, as shown in Fig. 7, the engaging ring 61 is arranged on the outer circumferential surface of the engagement body 60 while being fitted onto the engaging tubular part 601. Specifically, the engaging ring 61 is arranged between the engaging annular part 600 and the flange part 602 in the center axis direction X. Therefore, the length in the center axis direction X of the engaging ring 61 is shorter than the length in the center axis direction X of the engaging tubular part 601. An outer diameter of the engaging ring 61 is substantially the same as the outer diameter of the engaging annular part 600 and the outer diameter of the one side end in the center axis direction X of the flange part 602 of the engagement body 60.

The outer circumferential surface of the engaging ring 61 is provided with a ring projection 61A configured to fit to the plunger receiving recess 22B in a recess/projection way, and a ring recess 61B configured to fit to the plunger-receiving projection 22A in a recess/projection way. As shown in Fig. 6, the circumferential position of the ring recess 61B is substantially the same as the circumferential position of the body engagement part 60C. Therefore, two pairs of the ring recesses 61B are provided such that those of one pair are arranged in the first radial direction Y1 and those of another pair are arranged in the second radial direction Y2.

The elastic body 62 is configured to be elastically deformed in the circumferential direction. The elastic body 62 in this embodiment is an O-ring having an annular shape. Specifically, the elastic body 62 of this embodiment is formed of an oil bleed silicone rubber made by kneading oil into a silicone rubber. The elastic body 62 can be formed of an oil bleed silicone rubber material, such as a fluorine rubber, a nitrile rubber, or a silicone rubber.

The elastic body 62 is arranged between the engagement body 60 and the engaging ring 61. In this embodiment, one elastic body 62 is arranged between the engagement body 60 and the engaging ring 61 in the center axis direction X. Specifically, the elastic body 62 is arranged between one side end in the center axis direction X of the engaging annular part 600 and the other side end in the center axis direction X of the engaging ring 61, and another elastic body 62 is arranged between the one side end in the center axis direction X of the engaging ring 61 and the other side end in the center axis direction X of the flange part 602. Thus, in this embodiment, two elastic bodies 62 are arranged between the engagement body 60 and the engaging ring 61 respectively on one side and the other side in the center axis direction X. Each of the elastic bodies 62 abuts on the engagement body 60 and the engaging ring 61.

A length in the center axis direction X of the engaging member 6 is shorter than the distance between the partition part 21 and the plunger-receiving projection 22A in the center axis direction X. The length in the radial direction of the engaging member 6 is larger than the inner diameter of the partition part 21.

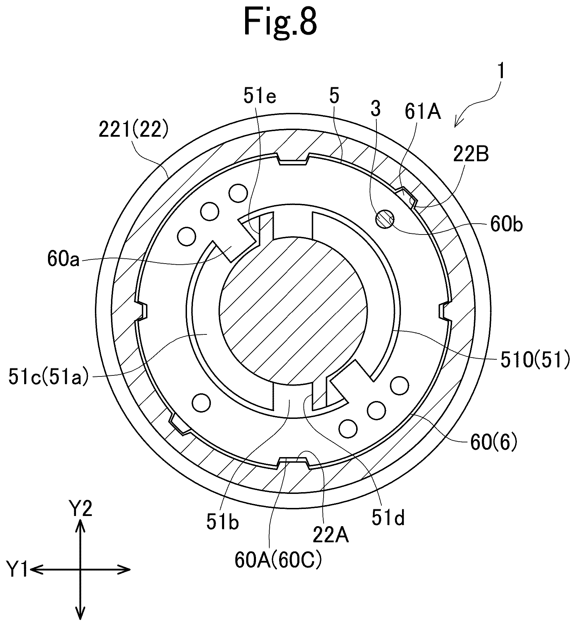

The engaging member 6 engages with the plunger engagement part 514. Specifically, when the plunger base part 513 is inserted into the engaging member 6, the radially inward projection 60a is inserted into the groove 51a so that the engaging member 6 engages with the plunger engagement part 514. In this embodiment, the radially inward projection 60a inserted into the axial groove 51b is moved to the circumferential groove 51c, and then the engaging member 6 and the plunger body 510 are rotated with respect to each other around the center axis O. Specifically, the engagement body 60 is rotated around the center axis O with respect to the plunger 51. Thus, as shown in Fig. 8, the radially inward projection 60a is arranged on the side of the circumferential other side end 51e of the circumferential groove 51c. Thus, the engaging member 6 engages with the plunger 51.

As shown in Fig. 1, the engaging member 6 in engagement with the plunger 51 is received in the plunger receiving part 22. This engaging member 6 compresses the plunger urging part 3 in the center axis direction X without torsion released. Here, in a state in which the plunger urging part 3 is twisted around the center axis O, the other side end in the center axis direction X of the plunger urging part 3 is inserted into the insertion hole provided in the trigger mounting base part 231, and one side end in the center axis direction X is inserted into the insertion hole 60b of the engaging annular part 600. In contrast to this, the engaging member 6 is received in the plunger-other-side receiving part 221, in a state in which the body engagement part 60C of the engagement body 60 and the ring recess 61B of the engaging ring 61 are fitted to the plunger-receiving projection 22A of the plunger-other-side receiving part 221 in a recess/projection way, and the ring projection 61A of the engaging ring 61 and the plunger receiving recess 22B of the plunger-other-side receiving part 221 are fitted in a recess/projection way. Therefore, by the engaging member 6 unable to rotate around the center axis O, the torsion of the plunger urging part 3 is prevented from being released. Also, by the engaging member 6 moving to the other side in the center axis direction X within the plunger-other-side receiving part 221, the plunger urging part 3 is compressed in the center axis direction X by the engaging member 6 and the trigger mounting base part 231. Further, by the trigger engagement part 515 inserted into the through hole formed by the trigger mounting base part 231, the rib 516 abuts on the trigger mounting base part 231 from the other side in the center axis direction X, as shown in Fig. 1. Thus, the engaging member 6 holds the plunger urging part 3 in a twisted state around the center axis O and a compressed state in the center axis direction X.

Also, in a state in which the engaging member 6 is received in the plunger-other-side receiving part 221, the elastic bodies 62 are arranged while being radially separated from the inner circumferential surface of the plunger-other-side receiving part 221.

The above is a description on the configuration of the injection device 1 according to the first embodiment. Next, the description will be made on how to use this injection device 1.

As shown in Fig. 1, the injection device 1 of this embodiment is held in an initial state before use. In this initial state, the injection needle 503 is received within the housing 2. Therefore, the barrel 50 is held in the first state. In the initial state, a non-illustrated cap is attached to one side in the center axis direction X of the housing 2, and the injection needle 503 does not protrude outside the housing 2. Therefore, when using the injection device 1, the cap is first removed from the housing 2.

Next, the injection needle 503 is made to protrude from one side in the center axis direction X of the housing 2, and the barrel 50 is moved from the first state to the second state. Accordingly, the trigger part 23 is unlocked, and the trigger body part 23A is pushed to one side in the center axis direction X. Specifically, the trigger lock operation part 23a and the trigger body part 23A are rotated around the center axis direction X with respect to the trigger mounting part 230 to have a circumferential position of the release groove 23D aligned with the circumferential position of the lock step part 233. Here, as the trigger body part 23A rotates around the center axis direction X, the thickness of the flat plate part 23G is arranged in a direction that matches the direction in which the bifurcated portions of the trigger engagement part 515 face each other. Therefore, the trigger engagement part 515 can be bent to have the bifurcated portions approaching each other. Then, the trigger body part 23A is pushed to one side in the center axis direction X. Thus, the lock step part 233 is inserted into the release groove 23D, and the inner skirt part 23F abuts on the rib 516. Thus, the bifurcated portions of the trigger engagement part 515 are bent so that they approach each other, and engagement between the plunger 51 and the trigger part 23 is released. Thus, the urging of the plunger urging part 3 held in a compressed state is released.

As shown in Fig. 11, the engaging member 6 and the syringe 5 including the plunger 51 engaging with the engaging member 6 move to one side in the center axis direction X due to the urging of the released plunger urging part 3. Here, the urging force of the barrel urging part 4 is set weaker than the urging force of the plunger urging part 3. Therefore, due to the urging of the plunger urging part 3, the syringe 5 first moves to one side in the center axis direction X while compressing the barrel urging part 4 via the engaging member 6. Specifically, the barrel 50 moves to one side in the center axis direction X until the cover part 50A abuts on the barrel step part 20D while compressing the barrel urging part 4. That is, the barrel urging part 4 is compressed by the plunger urging part 3 via the syringe 5. Note that the cover recess 50D and the barrel projection 20A are held in engagement with each other. Therefore, the barrel 50 is prevented from rotating around the center axis direction X. Then, as the syringe 5 moves to one side in the center axis direction X, the injection needle 503 protrudes outward from one side in the center axis direction X of the housing 2. Thus, the barrel 50 moves from the first state to the second state. Then, when the cover part 50A abuts on the barrel step part 20D, the movement of the barrel 50 to one side in the center axis direction X is restricted.

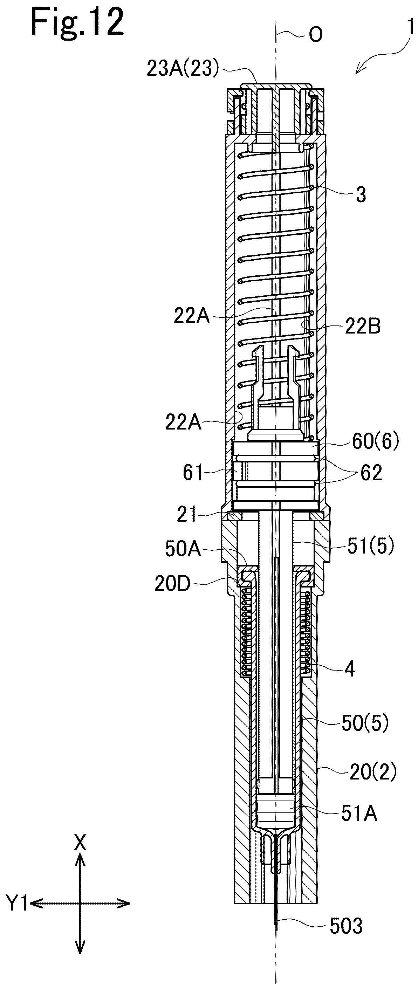

The engaging member 6 and the plunger 51 move further to one side in the center axis direction X due to the urging force of the plunger urging part 3. Specifically, as shown in Figs. 11 and 12, the plunger 51 moves within the barrel 50 with the other side in the center axis direction X, which is the proximal end side of the barrel 50, as a starting point, and one side in the center axis direction X, which is the distal end side provided with the injection needle 503, as a terminal end point. Thus, as the plunger 51 moves within the barrel 50, the piston 51A pushes the drug solution out of the barrel 50 from the injection needle 503. Thus, the drug solution in the barrel 50 can be discharged. The piston 51A moves to the distal end which is one side end in the center axis direction X of the barrel 50.

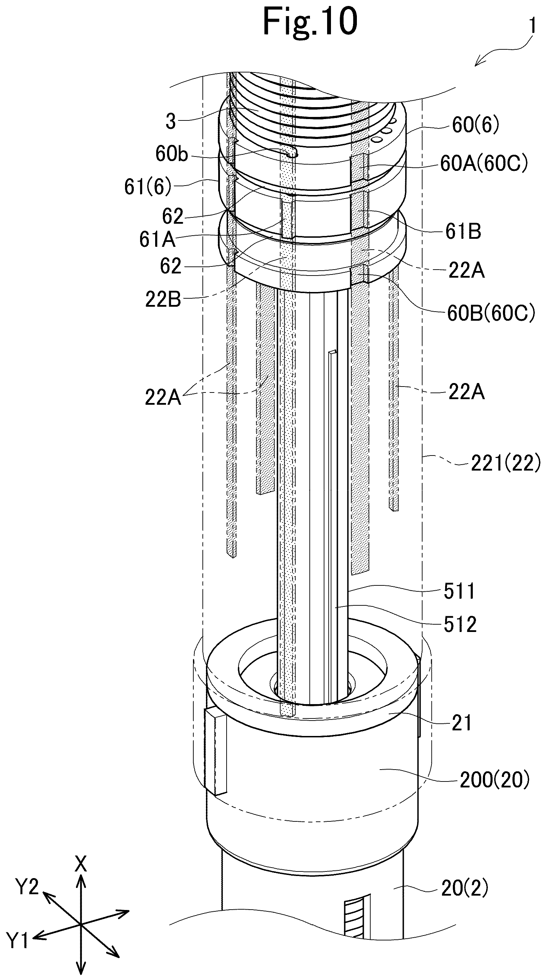

Here, in addition to the function of urging the syringe 5 and the engaging member 6 to one side from the other side in the center axis direction X, the plunger urging part 3 of this embodiment urges the syringe 5 and the engaging member 6, allowing them to rotate around the center axis O. Then, the other side end in the center axis direction X of the plunger urging part 3 is inserted into the insertion hole provided in the trigger mounting base part 231 from one side in the center axis direction X. Also, as shown in Figs. 8 and 10, one side end in the center axis direction X of the plunger urging part 3 is inserted into the insertion hole 60b. On the other hand, the annular recess 60A and the flange recess 60B are fitted to the plunger-receiving projection 22A in a recess/projection way. Therefore, the engagement body 60 cannot rotate around the center axis O. Therefore, in a state in which the body engagement part 60C and the plunger-receiving engagement part are held in engagement with each other, the torsion of the plunger urging part 3 is not released, and the engaging member 6 moves within the plunger-other-side receiving part 221 to one side in the center axis direction X without rotating around the center axis O. As shown in Figs. 8 and 10, the ring recess 61B and the plunger-receiving projection 22A, and the ring projection 61A and the plunger receiving recess 22B respectively fit to each other in a recess/projection way.

Also, as shown in Fig. 9, the axial movement protrusions 512 are respectively fitted in the axial movement grooves 50C. Therefore, the plunger 51 is configured to be unable to rotate around the center axis O. Thus, the plunger body 510 also moves within the plunger receiving part 22 to one side in the center axis direction X without rotating around the center axis O. Therefore, as shown in Fig. 8, the engaging member 6 and the plunger body 510 move to one side in the center axis direction X while the radially inward projection 60a and the groove 51a are held in engagement with each other.

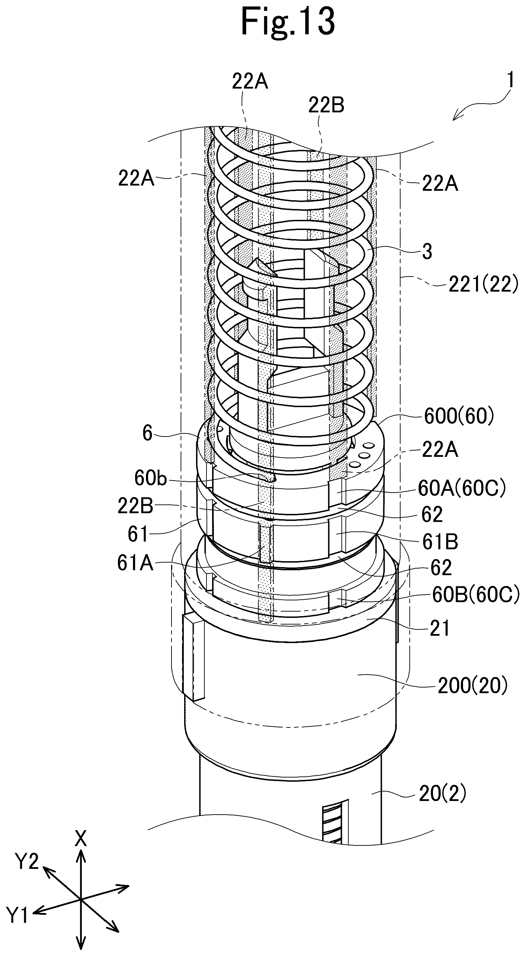

As described above, the length in the center axis direction X of the engaging member 6 is shorter than the distance between the partition part 21 and the plunger-receiving projection 22A in the center axis direction X. As shown in Figs. 12 and 13, when the engaging member 6 and the plunger body 510 move to one side of the center axis direction X until the piston 51A moves to one side end in the center axis direction X, which is the terminal end point of the barrel 50, the engaging member 6 is arranged between the partition part 21 and the plunger-receiving projection 22A. Also, fitting in a recess/projection way of the annular recess 60A, the flange recess 60B, and the ring recess 61B to the plunger-receiving projection 22A is eliminated. That is, the engagement between the body engagement part 60C and the plunger receiving part 22 is eliminated. Thus, since the engagement body 60 is brought into a rotatable state around the center axis O, the torsion of the plunger urging part 3 can be released.

On the other hand, even after the plunger body 510 moves to one side in the center axis direction X, the axial movement protrusion 512 remains fitted in the axial movement groove 50C. Also, as shown in Fig. 13, the ring projection 61A remains fitted to the plunger receiving recess 22B in a recess/projection way. Thus, while the engagement body 60 is rotatable around the center axis O, the engaging ring 61 and the plunger body 510 remain unable to rotate around the center axis O.

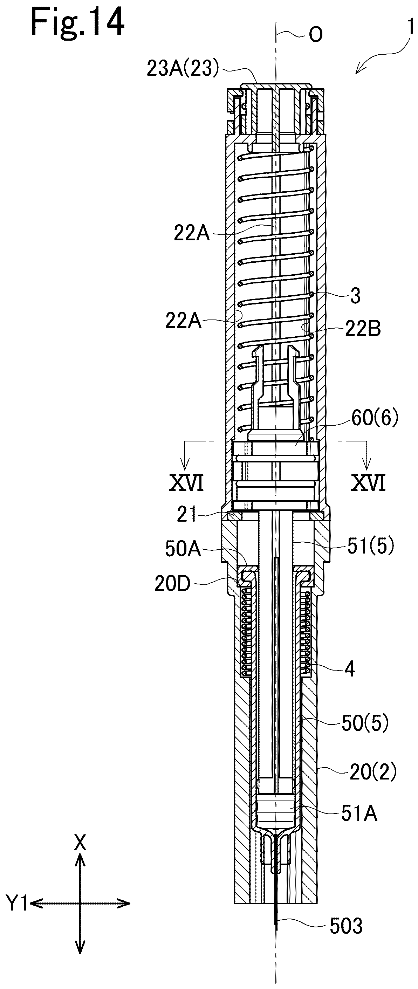

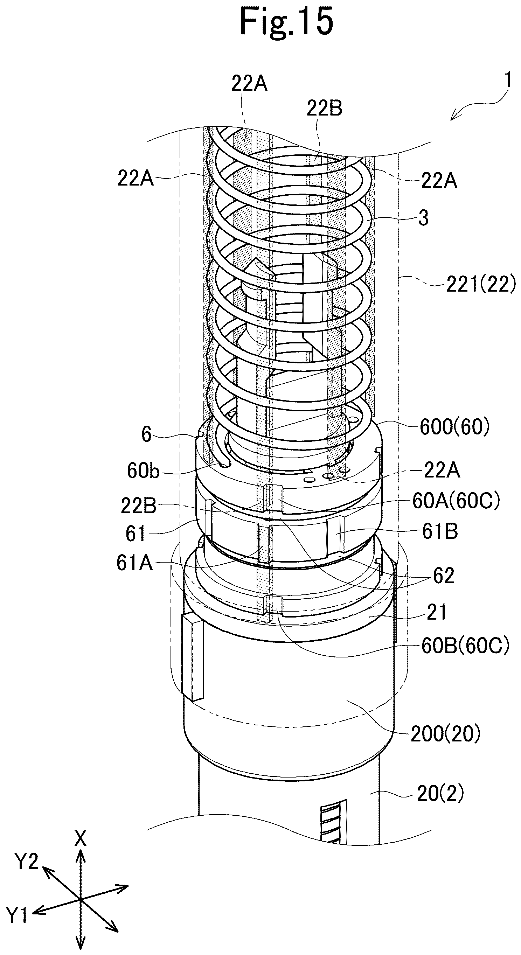

As shown in Figs. 13 and 15, one side end in the center axis direction X of the plunger urging part 3 is fixed to the fixing part of the engaging annular part 600. Specifically, the one side end in the center axis direction X of the plunger urging part 3 is inserted into the insertion hole 60b. Therefore, when the engagement between the body engagement part 60C and the plunger receiving part is eliminated, the engagement body 60 is urged to rotate around the center axis O due to the torsion of the released plunger urging part 3. Thus, the engagement body 60 transitions from the state before rotation shown in Figs. 12 and 13 to the state after rotation shown in Figs. 14 and 15.

On the other hand, as described above, the engaging ring 61 and the plunger body 510 remain unable to rotate around the center axis O. Therefore, the engagement body 60 rotates around the center axis O with respect to the engaging ring 61 and the plunger body 510, which are unable to rotate. Therefore, in this embodiment, the engagement body 60 is configured as an engaging rotation part that rotates around the center axis O with respect to the plunger 51, and the engaging ring 61 is configured as an engaging non-rotation part that does not rotate around the center axis O with respect to the engaging rotation part.

Here, as shown in Fig. 12, the elastic bodies 62 are arranged between the engagement body 60 and the engaging ring 61 while abutting on the engagement body 60 and the engaging ring 61. Therefore, the engagement body 60 rotates around the center axis O while allowing the elastic bodies 62 to elastically deform the elastic body 62 in the circumferential direction. Also, the elastic force of each of the elastic bodies 62 acts on the engagement body 60 and the engaging ring 61, and the engagement body 60 rotates around the center axis O while receiving the elastic force of each of the elastic bodies 62 in an elastically deformed state. Thus, the elastic force of each of the elastic bodies 62 becomes rotational resistance which is a resistance force in the opposite direction to the direction in which the engagement body 60 rotates, and the rotation speed of the engagement body 60 is restricted by this rotational resistance. Therefore, the elastic bodies 62 are arranged between the engaging rotation part and the engaging non-rotation part, and are configured as rotation restricting parts that restrict the rotation speed of the engaging rotation part with respect to the engaging non-rotation part.

In this embodiment, two elastic bodies 62 are arranged between the engagement body 60 and the engaging ring 61 on one side and the other side in the center axis direction X. Therefore, the engagement body 60 of this embodiment rotates around the center axis O while receiving rotational resistance from the elastic bodies 62 on one side and the other side in the center axis direction X.

Also, since the rotation speed of the engagement body 60 is restricted, the movement of the radially inward projection 60a from the side of the circumferential other side end 51e toward the circumferential one side end 51d of the circumferential groove 51c becomes slow. Therefore, while the radially inward projection 60a continues to move along the circumferential groove 51c, a state in which the engaging member 6 and the plunger 51 are engaged with each other is maintained. Thus, the injection needle 503 is maintained in a state in which it protrudes from the housing 2. In this embodiment, the injection needle 503 remains protruding from the housing 2 for 3 to 5 seconds.

Thus, a rotation restricting mechanism that restricts the rotation speed around the center axis O of the engaging member 6 with respect to the plunger 51 is configured. In this embodiment, the rotation restricting mechanism includes the engaging ring 61 as an engaging non-rotation part, and the elastic bodies 62 as rotation restricting parts, and is configured to restrict the rotation speed of the engagement body 60 with respect to the engaging ring 61.

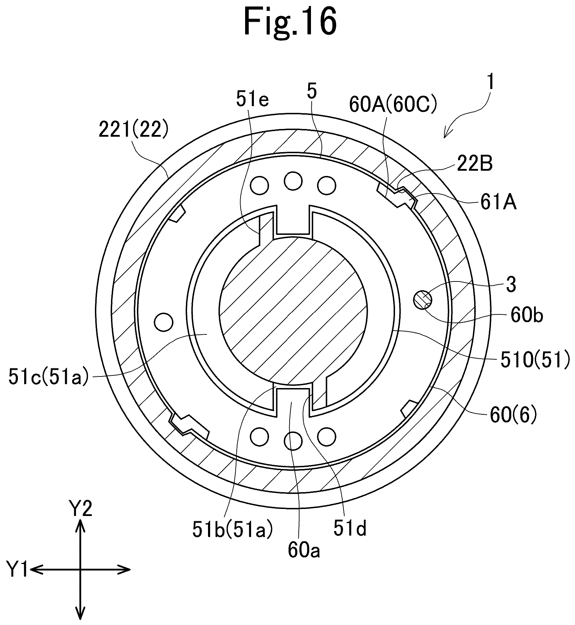

As shown in Fig. 16, when the radially inward projection 60a moves toward the circumferential one side end 51d of the circumferential groove 51c, the axial groove 51b and the radially inward projection 60a are arranged to have their circumferential positions aligned with each other. Thus, the engagement between the engaging member 6 and the plunger body 510 is released.

By releasing the engagement between the engaging member 6 and the plunger body 510, the urging by the plunger urging part 3 to the syringe 5 is released. Therefore, the barrel urging part 4 compressed by the plunger urging part 3 via the syringe 5 is released. Thus, as shown in Fig. 17, the syringe 5 moves to the other side in the center axis direction X due to the urging by the barrel urging part 4. Here, as shown in Fig. 16, when the engagement between the engaging member 6 and the plunger 51 has been released, the radially inward projection 60a and the axial groove 51b are arranged in a state in which their circumferential positions are aligned with each other. Therefore, while the axial groove 51b moves to the other side in the center axis direction X with respect to the radially inward projection 60a, the syringe 5 moves to the other side in the center axis direction X. Therefore, as shown in Fig. 17, the barrel 50 moves from the second state to the first state, so that the injection needle 503 can be received in the housing 2. When the cover part 50A abuts on the partition part 21 from one side in the center axis direction X, the syringe 5 stops.

Therefore, as shown in Figs. 14, 15, and 16, the barrel 50 is received in the barrel receiving part 20 while the injection needle 503 protrudes from one side in the center axis direction X of the housing 2, and the plunger 51 and the engaging member 6 rotate with respect to each other around the center axis O, while the plunger 51 is positioned at the terminal end point due to the urging force of the plunger urging part 3. Thus, an engagement releasing mechanism for releasing the engagement between the plunger 51 and the engaging member 6 is configured. In this embodiment, the engagement body 60 rotates with respect to the plunger body 510 due to the torsion of the plunger urging part 3, and the radially inward projection 60a and the axial groove 51b are arranged to have their circumferential positions aligned with each other. Thus, the engagement between the plunger body 510 and the engaging member 6 is released. Therefore, in this embodiment, the plunger urging part 3 that urges the engagement body 60 to rotate it around the center axis O, and the axial groove 51b together constitute the engagement releasing mechanism.

As described above, according to this embodiment, when the engagement body 60 rotates around the center axis O with respect to the plunger body 510, the elastic force of each of the elastic bodies 62 restricts the rotation speed of the engagement body 60 as rotation resistance, so the time until the engagement between the engaging member 6 and the plunger 51 is released can be lengthened. Thus, the injection needle 503 that has discharged the drug solution can be maintained in a state in which it protrudes from the housing 2. In this embodiment, the injection needle 503 can be maintained in a state in which it protrudes from the housing 2 for 3 to 5 seconds. Thus, for example, it is possible to prevent the injection needle 503 pierced into the patient's skin from falling out of the skin and leaking the drug solution from the pierced site.

Also, in this embodiment, the plunger 51 is configured to be unable to rotate around the center axis O, the engagement body 60 is configured to rotate around the center axis O, and the plunger urging part 3 gives an urging force to the engagement body 60 to rotate around the center axis O. Also, in the engagement releasing mechanism, the engagement body 60 rotates with respect to the plunger 51 due to the urging force of the plunger urging part 3 to release the engagement between the engagement body 60 and the plunger 51, and the rotation restricting mechanism restricts the rotation speed of the engagement body 60 rotating around the center axis O of the plunger urging part 3 due to the urging force. Thus, by the rotation restricting mechanism that restricts the rotation speed of the engagement body 60 with respect to the engaging ring 61, the time until the engagement between the engagement body 60 and the plunger 51 is released by the engagement releasing mechanism can be lengthened.

In this embodiment, the plunger receiving recess 22B is formed over the entire area in the center axis direction X of the plunger-other-side receiving part 221, and the ring projection 61A of the engaging ring 61 engages with the plunger receiving recess 22B. Therefore, the engaging ring 61 cannot rotate around the center axis O. Also, the elastic bodies 62 are arranged between the engagement body 60 and the engaging ring 61. Thus, the engaging ring 61 and the elastic bodies 62 can be prevented from rotating together due to the rotation of the engagement body 60, and a mechanism for restricting the rotation speed of the engagement body 60 can be actuated reliably.

Also, the elastic bodies 62 of this embodiment each are composed of an oil bleed silicone rubber. Therefore, since oil kneaded into the silicone oozes out to the surface of each of the elastic bodies 62, the rotation speed of the engagement body 60 can be adjusted, and as a result, the rotation speed of the engagement body 60 can be appropriately restricted by the elastic bodies 62.

Also, in this embodiment, the elastic bodies 62 are arranged between the engagement body 60 and the engaging ring 61 in the center axis direction X. Therefore, the elastic bodies 62 do not affect the movement of the syringe 5 and the engaging member 6 to one side in the center axis direction X due to the urging force of the plunger urging part 3. Further, in a state in which the engaging member 6 is received in the plunger-other-side receiving part 221, the elastic bodies 62 are arranged while being radially separated from the inner circumferential surface of the plunger-other-side receiving part 221. Thus, when the engaging member 6 moves to one side in the center axis direction X, the elastic body 62 abuts on the plunger-other-side receiving part 221 and prevents the movement of the engaging member 6 to one side in the center axis direction X.

Also, in this embodiment, the engaging ring 61 is arranged between the engaging annular part 600 and the flange part 602 in the center axis direction X, and the elastic bodies 62 are arranged between the engagement body 60 and the engaging ring 61 in the center axis direction X. Therefore, when the engagement body 60 rotates around the center axis O, it is possible to prevent the occurrence of a situation in which the elastic bodies 62 are separated away from the engagement body 60 and hence the rotation speed of the engagement body 60 is not restricted.

Also, in this embodiment, the two elastic bodies 62 are arranged between the engagement body 60 and the engaging ring 61 on one side and the other side in the center axis direction X. Therefore, the engagement body 60 of this embodiment rotates around the center axis O while receiving rotational resistance from the elastic bodies 62 on one side and the other side in the center axis direction X. Therefore, the rotation speed of the engagement body 60 can be further restricted.

The barrel-one-side receiving part 201 of this embodiment is provided with a penetrating part 20C that penetrates radially. Therefore, the remaining amount of the drug solution in the barrel 50 received in the housing 2 can be checked.

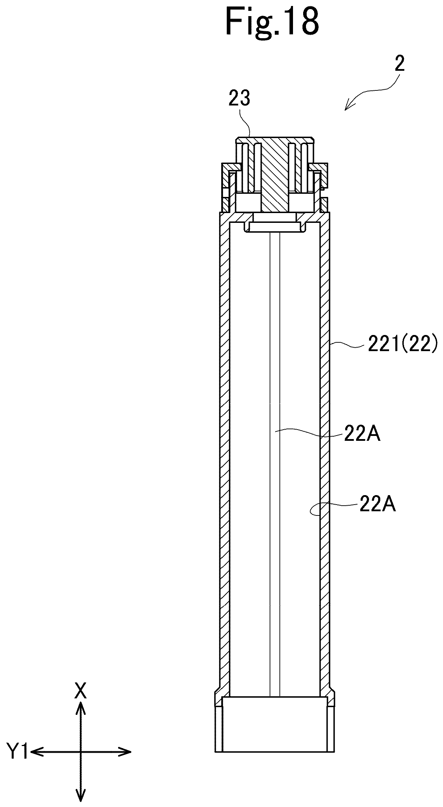

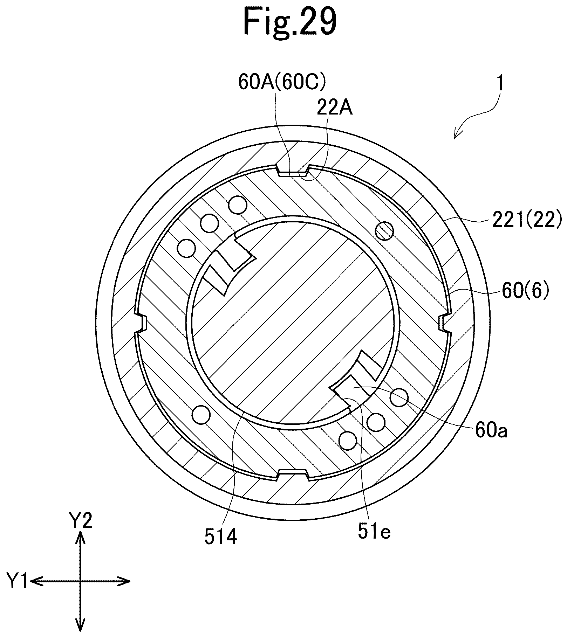

Next, with reference to Figs. 18 to 30, the description on the injection device 1 according to a second embodiment will be given. When the description will be given on the second embodiment, the description for the same configuration, usage, and effects as those of the first embodiment will be omitted.

As shown in Fig. 18, the plunger-receiving engagement part of the second embodiment includes only the plunger-receiving projection 22A. Therefore, the plunger-receiving engagement part of the second embodiment does not include the plunger receiving recess 22B. Also, the plunger-receiving projection 22A of the second embodiment is formed over the entire area in the center axis direction X of the plunger-other-side receiving part 221. Two pairs of the plunger-receiving projections 22A are provided such that those of one pair are arranged in the first radial direction Y1 and those of another pair are arranged in the second radial direction Y2.

The plunger urging part 3 in the second embodiment is a compression coil spring wound around the center axis O. Therefore, unlike the first embodiment, the plunger urging part 3 of the second embodiment urges the syringe 5 and the engaging member 6 only from the other side to one side in the center axis direction X.

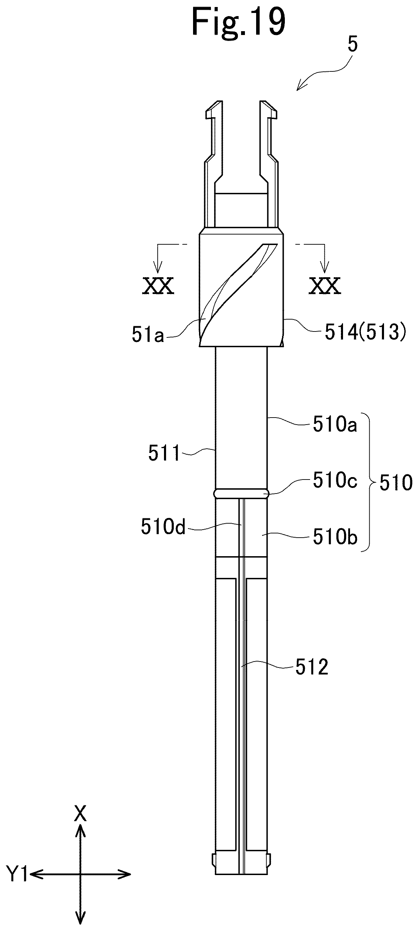

As shown in Fig. 19, the plunger body 510 in the second embodiment includes a plunger body part 510a, a plunger ring 510b formed in an annular shape, and an elastic body 510c. Also, the plunger body part 510a includes a rod 511 and a plunger base part 513.

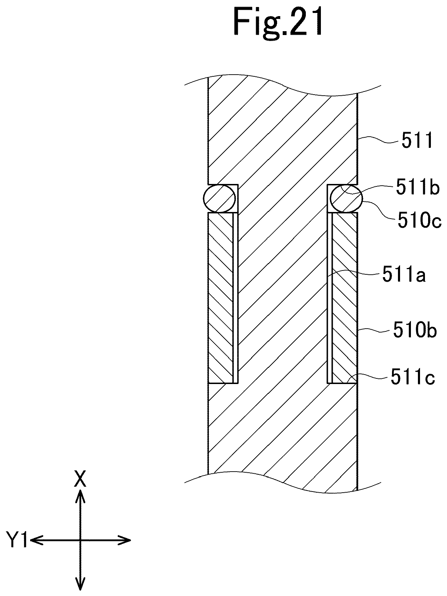

As shown in Fig. 21, the rod 511 is configured to have a middle portion 511a with a smaller diameter than the other portions in the center axis direction X. Accordingly, rod step parts 511b, 511c extending radially are formed between the middle portion 511a in the center axis direction X and the portions arranged on one side and the other side of the middle portion 511a in the center axis direction X. These rod step parts 511b, 511c are flat surfaces that extend along the radial direction and in the circumferential direction. Also, as shown in Fig. 19, the axial movement protrusion 512 of the second embodiment is formed on one side in the center axis direction X of the middle portion 511a in the center axis direction X, of the rod 511.

The plunger ring 510b and the elastic body 510c each formed in an annular shape are arranged in the middle portion 511a in the center axis direction X of the rod 511. Therefore, as shown in Fig. 21, the inner diameter of the plunger ring 510b is larger than the outer diameter of the middle portion 511a in the center axis direction X of the rod 511. On the other hand, the outer diameter of the plunger ring 510b is substantially the same as the outer diameter of the portions other than the middle portion 511a in the center axis direction X of the rod 511. In this embodiment, one side end in the center axis direction X of the plunger ring 510b abuts on the rod step part 511c.

As shown in Fig. 19, a radially outward protrusion 510d protruding radially outward is formed on the outer circumferential surface of the plunger ring 510b. This radially outward protrusion 510d is configured identically to the axial movement protrusion 512 formed on the rod 511. Therefore, the radially outward protrusion 510d is configured so that it can be fitted in the axial movement groove 50C. The radially outward protrusion 510d is arranged to have a circumferential position substantially aligned with the circumferential position of the axial movement protrusion 512 in the circumferential direction. Thus, the radially outward protrusion 510d is continuous with the axial movement protrusion 512 in the center axis direction X.

The elastic body 510c is an O-ring that can be elastically deformed in the circumferential direction. The elastic body 510c of this embodiment is formed of an oil bleed silicone rubber. Similar to the first embodiment, the elastic body 510c can be formed of an oil bleed silicone rubber material, such as a fluorine rubber, a nitrile rubber, or a silicone rubber.

As shown in Figs. 19 and 21, the elastic body 510c is arranged between the plunger body part 510a and the plunger ring 510b. The elastic body 510c of this embodiment is arranged between the rod 511 and the plunger ring 510b in the center axis direction X. Specifically, the elastic body 510c abuts on the rod step part 511b and the other side end in the center axis direction X of the plunger ring 510b.

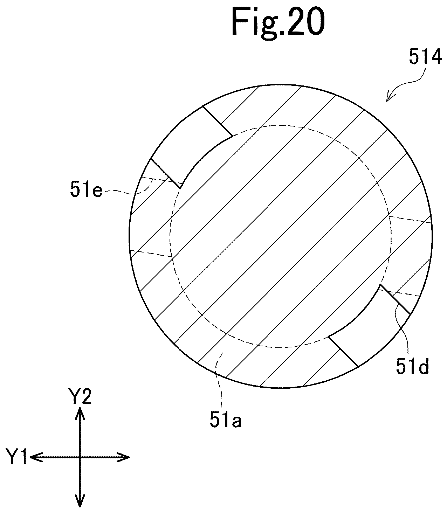

As shown in Fig. 19, the groove 51a recessed radially inward is formed in the outer circumferential surface of the plunger engagement part 514 of the second embodiment, similar to the first embodiment. Here, the groove 51a in the second embodiment is configured as a spiral groove 51a extending in the circumferential direction from one side end to the other side in the center axis direction X of the plunger engagement part 514. Also, as shown in Fig. 20, the spiral groove 51a includes the circumferential one side end 51d, which is one side end in the circumferential direction, and the circumferential other side end 51e, which is the other side end in the circumferential direction. Also, the circumferential one side end 51d is arranged on the other side in the center axis direction X, and the circumferential other side end 51e is arranged on one side in the center axis direction X.

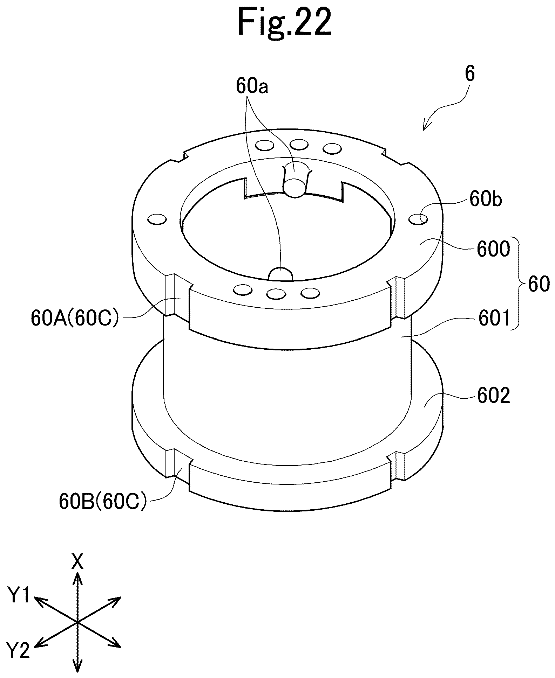

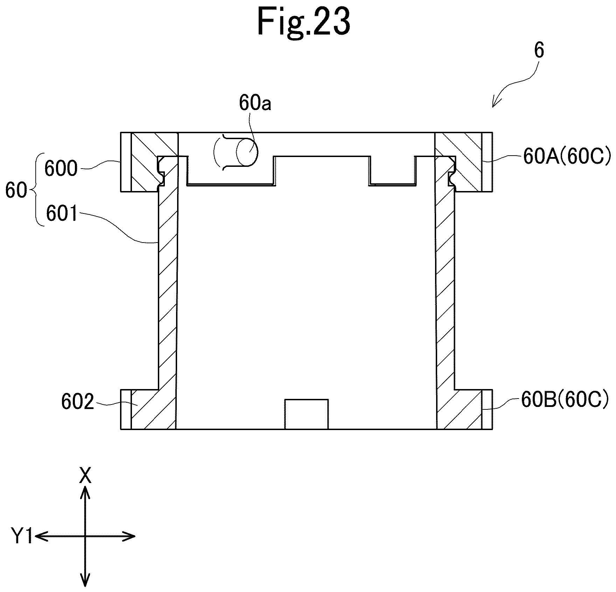

As shown in Figs. 22 and 23, the engaging member 6 in the second embodiment has only the engagement body 60. Therefore, unlike the first embodiment, the engaging member 6 of the second embodiment does not include the engaging ring 61 and the elastic body 62. Also, the flange part 602 in the second embodiment has the same amount of protrusion on one side as that on the other side in the center axis direction X. Further, the radially inward projection 60a of the second embodiment is configured to be inserted into the spiral groove 51a.

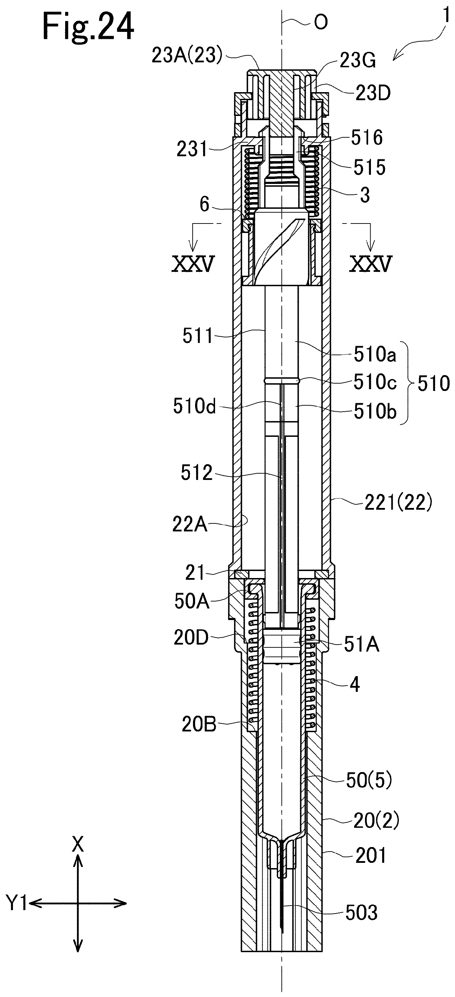

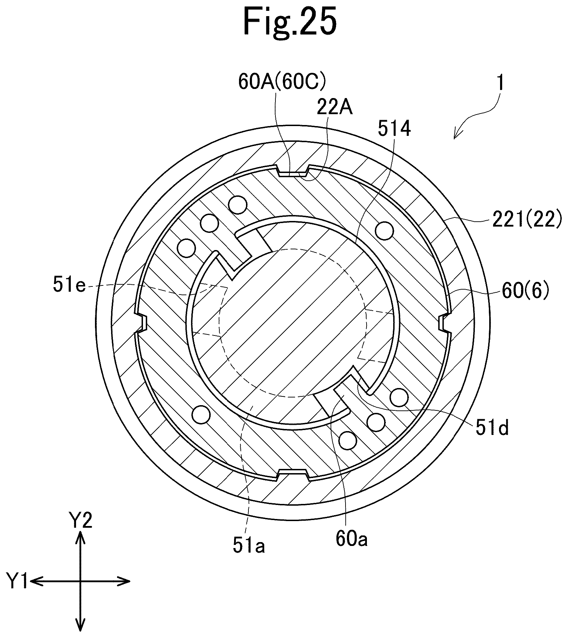

As shown in Fig. 24, the engaging member 6 engages with the plunger engagement part 514. In this embodiment, the engaging member 6 and the plunger body 510 rotate with respect to each other around the center axis O so that the radially inward projection 60a inserted into the spiral groove 51a from one side in the center axis direction X moves from the side of the circumferential other side end 51e toward the circumferential one side end 51d. Thus, as shown in Fig. 25, the radially inward projection 60a is arranged on the side of the circumferential one side end 51d of the spiral groove 51a.

As described above, the plunger urging part 3 of the second embodiment is configured to urge the syringe 5 and the engaging member 6 from the other side toward one side in the center axis direction X. Therefore, as shown in Fig. 24, the engaging member 6 received in the plunger-other-side receiving part 221 compresses the plunger urging part 3 in the center axis direction X. Further, as shown in Fig. 25, in a state in which the body engagement part 60C of the engagement body 60 and the plunger-receiving projection 22A of the plunger-other-side receiving part 221 are fitted to each other in a recess/projection way, the engaging member 6 is received in the plunger other side receiving part 221.

Next, the usage of the injection device 1 in the second embodiment will be described. Here, in describing the usage of the injection device 1 of the second embodiment, it is similar to the first embodiment until the injection needle 503 protrudes from the initial state. Therefore, the description for this part is omitted.

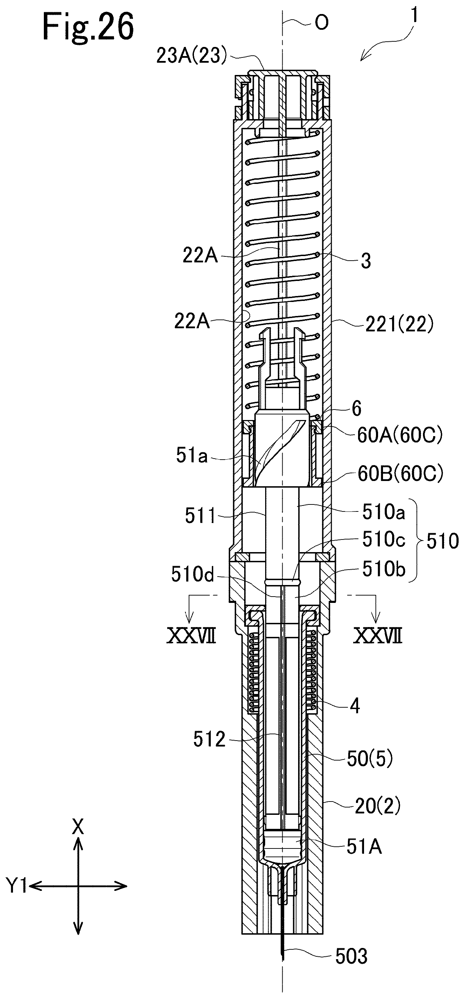

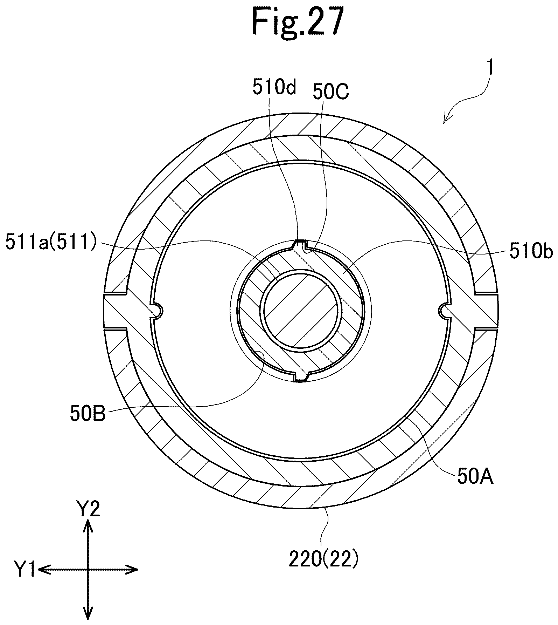

As shown in Figs. 26 and 27, in a state in which the piston 51A has moved to the distal end which is one side end in the center axis direction X of the barrel 50, the radially outward protrusion 510d is fitted in the axial movement groove 50C. Therefore, the plunger ring 510b is configured to be unable to rotate around the center axis O.

On the other hand, due to the urging force of the plunger urging part 3, the engaging member 6 tries to move further to one side in the center axis direction X. Here, in the second embodiment, the plunger-receiving projection 22A is formed over the entire area in the center axis direction X of the plunger-other-side receiving part 221, and the body engagement part 60C of the engagement body 60 and the plunger-receiving projection 22A of the plunger-other-side receiving part 221 are fitted to each other in a recess/projection way. Therefore, the engaging member 6 cannot rotate around the center axis O. Thus, while the radially inward projection 60a moves from the side of the circumferential one side end 51d of the spiral groove 51a toward the circumferential other side end 51e, the engaging member 6 which cannot rotate around the center axis O moves to one side in the center axis direction X. Therefore, when the engaging member 6 moves to one side in the center axis direction X, the plunger body 510 rotates around the center axis O. The plunger body 510 in this embodiment rotates counterclockwise around the center axis O.

Then, when the plunger body 510 rotates, the rod 511 rotates around the center axis O with respect to the plunger ring 510b configured to be unable to rotate. Therefore, in this embodiment, the plunger body part 510a is configured as a plunger rotation part rotating about the center axis O with respect to the engaging member 6, and the plunger ring 510b is configured as a plunger non-rotation part that does not rotate around the center axis with respect to the plunger rotation part.

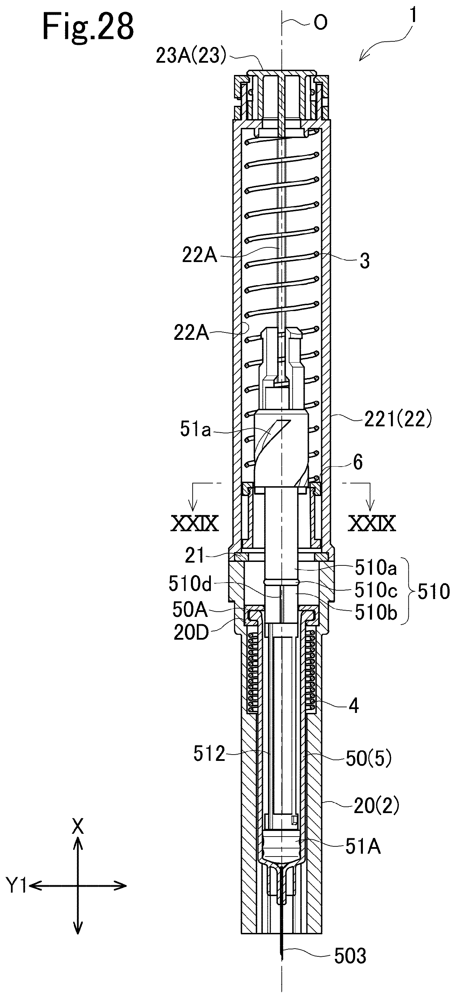

Here, as shown in Fig. 26, the elastic body 510c is arranged between the plunger body part 510a and the plunger ring 510b. Also, the elastic body 510c abuts on the rod step part 511b and the other side end in the center axis direction X of the plunger ring 510b. Therefore, as shown in Fig. 28, the plunger body part 510a rotates around the center axis O while elastically deforming the elastic body 510c in the circumferential direction. Also, the elastic force of the elastic body 510c acts on the plunger body part 510a and the plunger ring 510b, and the plunger body part 510a rotates around the center axis O while receiving the elastic force of the elastic body 510c in an elastically deformed state. Thus, the elastic force of the elastic body 510c becomes rotational resistance which is a resistance force in the opposite direction to the direction in which the plunger body part 510a rotates, and hence the rotation speed of the plunger body part 510a is restricted by this rotational resistance. Therefore, the elastic body 510c is arranged between the plunger rotation part and the plunger non-rotation part, and is configured as a rotation restricting part that restricts the rotation speed of the plunger rotation part with respect to the plunger non-rotation part.

Since the rotation speed of the plunger body part 510a is restricted, the movement of the radially inward projection 60a from the side of the circumferential one side end 51d toward the circumferential other side end 51e of the spiral groove 51a becomes slow. Therefore, while the radially inward projection 60a continues to move in the spiral groove 51a, the engaging member 6 and the plunger 51 are maintained in a state in which they are engaged with each other. Thus, the injection needle 503 is maintained in a state in which it protrudes from the housing 2.

Thus, the rotation restricting mechanism that restricts the rotation speed of the plunger 51 around the center axis O with respect to the engaging member 6 is configured. In this embodiment, the rotation restricting mechanism includes the plunger ring 510b, which is the plunger non-rotation part, and the elastic body 510c, which is the rotation restricting part, and restricts the rotation speed of the rod 511 with respect to the plunger ring 510b. Therefore, the rotation restricting mechanism restricts the rotation speed of the plunger 51.

When the radially inward projection 60a moves to the circumferential other side end 51e of the spiral groove 51a, the radially inward projection 60a is arranged at one side end in the center axis direction X of the plunger engagement part 514. Thus, the engagement between the radially inward projection 60a and the spiral groove 51a is released. Therefore, the engagement releasing mechanism of the second embodiment is configured so that the plunger 51 (specifically, the plunger body part 510a as the plunger rotation part) rotates around the center axis O with respect to the engaging member 6 and releases the engagement between the engaging member 6 and the plunger body part 510a, thereby releasing the engagement between the plunger 51 and the engaging member 6.

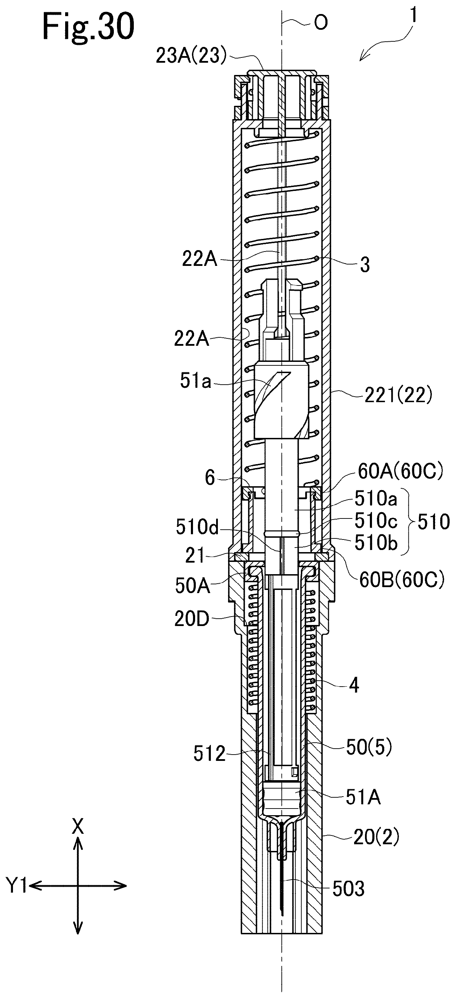

By releasing the engagement between the plunger 51 and the engaging member 6, the barrel urging part 4 which has been compressed by the plunger urging part 3 is released. Thus, as shown in Fig. 30, the syringe 5 moves to the other side in the center axis direction X due to the urging force of the barrel urging part 4. Thus, the injection needle 503 is received within the housing 2.

As described above, according to this embodiment, the time until the engagement between the plunger 51 and the engaging member 6 is released can be lengthened by restricting the rotation speed of the plunger 51. Thus, the injection needle 503 that has discharged the drug solution can be maintained in a state in which it protrudes from the housing 2.

Also, in this embodiment, the plunger body part 510a rotates around the center axis O with respect to the plunger ring 510b by the radially outward protrusion 510d inserted into the axial movement groove 50C. At that time, the rotation speed of the rod 511 can be restricted by the elastic force of the elastic body 510c arranged between the plunger body part 510a and the plunger ring 510b.

Also, according to the engaging member 6 in this embodiment, which moves to one side in the center axis direction X due to the urging force of the plunger urging part 3, the radially inward projection 60a moves along the spiral groove 51a to one side in the center axis direction X, so that the plunger body 510 rotates around the center axis O. Thus, the urging force directed to one side in the center axis direction X of the plunger urging part 3 is converted to the rotational force that rotates the plunger 51, which enables the rotation of the plunger 51.

The present invention is not limited to the above-described embodiments, and can be modified as appropriate within the scope of the invention.

In the second embodiment, the rotation around the center axis O of the engagement body 60 is restricted by fitting of the annular recess 60A of the engagement body 60 to the plunger-receiving projection 22A of the plunger-other-side receiving part 221 in a recess/projection way. However, the present invention is not necessarily limited to this, and can be configured such that, for example, the annular recess 60A projecting radially outward from the engagement body 60 is provided, to which the plunger receiving recess 22B of the plunger-other-side receiving part 221 is fitted in a recess/projection way, and thereby the rotation around the center axis O of the engagement body 60 is restricted.

In the above embodiments, the engaging member 6 includes the engaging rotation part and the engaging non-rotation part. However, the present invention is not necessarily limited to this. For example, the engaging member 6 can include only the engaging rotation part. For example, it can be configured such that, in the arrangement in which the engaging member 6 does not include the engaging ring 61 and the elastic body 62 while the partition part 21 includes an elastic body on the other side in the center axis direction X, the engagement body 60 abuts on the elastic body of the partition part 21 when the fitting in a recess/projection way between the annular recess 60A of the engagement body 60 and the plunger-receiving projection 22A of the plunger-other-side receiving part 221 is released. In this case, the engagement body 60 abuts on the elastic body in the center axis direction X.

In the above embodiment, the elastic body 62 is arranged between the engagement body 60 and the engaging ring 61 in the center axis direction X. However, the present invention is not necessarily limited to this. For example, the elastic body 62 can be arranged between the engagement body 60 and the engaging ring 61 in the radial direction. The same is also applicable to the elastic body 510c of the second embodiment.

For example, it is possible to change the time during which the injection needle 503 remains protruding from the housing 2 by changing the circumferential length of the circumferential groove 51c formed in the plunger engagement part 514.

In the above embodiments, the description was made on the case in which the barrel urging part 4 and the plunger urging part 3 are compression coil springs, but the present invention is not necessarily limited to this. For example, they can be rubber that is elastically deformable so as to extend in the center axis direction X.

In the above embodiments, either one of the engaging member 6 and the plunger 51 is fixed to be not rotatable around the axis, and the other is rotatable around the axis, in which the other is configured to rotate with respect to the fixed one. However, the present invention is not necessarily limited to this. For example, both the engaging member 6 and the plunger 51 can be configured to rotate around the center axis O.

The description was made on the above embodiments by taking, for example, the case in which the starting point is positioned on one side of the other side end in the center axis direction X of the barrel 50, and the terminal end point is positioned at one side end in the center axis direction X of the barrel 50. However, the present invention is not necessarily limited to this. For example, the starting point can be positioned at the other side end in the center axis direction X of the barrel 50. Also, the terminal end point can be positioned on the other side of one side end in the center axis direction X of the barrel 50. That is, as long as the positional relationship is such that the starting point is positioned on the other side in the center axis direction X of the barrel 50 and the terminal end point is positioned on one side in the center axis direction X of the barrel 50, the starting point can be positioned at the other side end in the center axis direction X of the barrel 50 and the terminal end point can be positioned at any portion other than the one side end in the center axis direction X of the barrel 50.

As described above, according to the present invention, the time until the engagement between the plunger and the engaging member is released can be lengthened, and thereby the injection can be reliably completed by maintaining the state in which the injection needle remains protruding from the housing for a while after the injection is completed.

The present invention is an injection device including: a barrel having a distal end with an injection needle; a syringe including a plunger that is inserted into the barrel so as to be reciprocable between a proximal end side of the barrel as a starting point and a distal end side of the barrel with the injection needle as a terminal end point; a housing having a tubular shape for receiving the syringe, the housing including a barrel receiving part on one side in a center axis direction so as to receive the barrel in a movable manner between a position at which the injection needle is received in the housing, and a position at which the barrel which has moved a certain distance from the position with the injection needle received toward one side to have the injection needle protruding from the one side of the housing, and a plunger receiving part on another side of the barrel receiving part to receive the plunger; a barrel urging part arranged in the barrel receiving part and configured to urge the barrel from the one side to the other side of the housing; an engaging member arranged in the plunger receiving part and configured to engage with the plunger; a plunger urging part configured to engage with the engaging member and urge the plunger from the starting point toward the terminal end point; an engagement releasing mechanism configured to release engagement between the plunger and the engaging member by rotating the plunger and the engaging member around a center axis with respect to each other in a state in which the barrel is received in the barrel receiving part with the injection needle protruding from the one side of the housing, and the plunger is positioned at the terminal end point due to an urging force of the plunger urging part; and a rotation restricting mechanism configured to restrict a rotation speed of relative rotation between the plunger and the engaging member in the engagement releasing mechanism.

According to the above configuration, injection of the drug solution is completed when the plunger is positioned at the terminal end point with the injection needle remaining protruding from the one side of the housing. Then, the engagement releasing mechanism allows the plunger and the engaging member to rotate with respect to each other in the state in which the plunger is positioned at the terminal end point to thereby release the engagement between the plunger and the engaging member. Here, since the injection device of the present invention includes the rotation restricting mechanism, it is possible to restrict the rotation speed of the relative rotation between the plunger and the engaging member when they rotate with respect to each other, and hence lengthen the time until the engagement between the plunger and the engaging member is released.

The present invention can be configured such that the engagement releasing mechanism is configured to release the engagement between the plunger and the engaging member by allowing the engaging member to rotate around the center axis with respect to the plunger, and the rotation restricting mechanism is configured to restrict the rotation speed of the engaging member.

According to the above configuration, the rotation restricting mechanism restricts the rotation speed of the engaging member, so that the time until the engagement between the plunger and the engaging member is released can be lengthened.

The present invention can be configured such that the engaging member includes an engaging rotation part that is configured to engage with the plunger and rotate around the center axis with respect to the plunger to release engagement with the plunger; the engagement releasing mechanism is configured to release the engagement between the plunger and the engaging member by allowing the engaging rotation part to rotate with respect to the plunger to release the engagement between the plunger and the engaging member; and the rotating restricting mechanism includes an engaging non-rotation part that does not rotate around the center axis with respect to the engaging rotation part, and a rotation restricting part that is arranged between the engaging rotation part and the engaging non-rotation part and is configured to restrict the rotation speed of the engaging rotation part with respect to the engaging non-rotation part to restrict the rotation speed of the engaging rotation part with respect to the engaging non-rotation part.

According to the above configuration, the rotation restricting mechanism restricts the rotation speed of the engaging rotation part with respect to the engaging non-rotation part, thereby restricting the rotation speed of the engaging rotation part with respect to the plunger, lengthening the time until the engagement between the plunger and the engaging rotation part is released, and lengthening the time until the engagement between the plunger and the engaging member is released.

The present invention can be configured such that the rotation restricting part is an elastic body arranged in an elastically deformed state between the engaging rotation part and the engaging non-rotation part.

According to the above configuration, since the rotation speed of the engaging rotation part with respect to the non-engagement part can be restricted by the elastic force of the rotation restricting part, the time until the engagement between the plunger and the engaging member is canceled can be lengthened.

The present invention can be configured such that the engagement releasing mechanism is configured to allow the plunger to rotate around the center axis with respect to the engaging member to release the engagement between the plunger and the engaging member; and the rotation restricting mechanism is configured to restrict the rotation speed of the plunger.

According to the above configuration, the rotation restricting mechanism restricts the rotation speed of the plunger, so that the time until the engagement between the plunger and the engaging member is released can be lengthened.

The present invention can be configured such that the plunger includes: a plunger rotation part that is configured to engage with the engaging member, and rotate around the center axis with respect to the engaging member to release the engagement; a plunger non-rotation part that does not rotate around the center axis with respect to the plunger rotation part; and a rotation restricting part that is arranged between the plunger rotation part and the plunger non-rotation part, and is configured to restrict the rotation speed of the plunger rotation part with respect to the plunger non-rotation part, in which the engagement releasing mechanism is configured to release the engagement between the engaging member and the plunger rotation part and by allowing the plunger rotation part to rotate with respect to the engaging member to release the engagement between the plunger and the engaging member, and the rotation restricting mechanism includes the plunger non-rotation part and the rotation restricting part, and is configured to restrict the rotation speed of the plunger rotation part with respect to the plunger non-rotation part.

According to the above configuration, the rotation restricting mechanism restricts the rotation speed of the plunger rotation part with respect to the plunger non-rotation part of the plunger, thereby restricting the rotation speed of the plunger rotation part with respect to the engaging member, lengthening the time until the engagement between the engaging member and the plunger rotation part is released, and lengthening the time until the engagement between the plunger and the engaging member is released.

The present invention can be configured such that the rotation restricting part is an elastic body arranged in an elastically deformed state between the plunger rotation part and the plunger non-rotation part.

According to the above configuration, since the rotation speed of the plunger rotation part with respect to the plunger non-rotation part can be restricted by the elastic force of the rotation restricting part, the time until the engagement between the plunger and the engaging member is released can be lengthened.

### REFERENCE SIGNS LIST

1: Injection device
2: Housing
20: Barrel receiving part
200: Barrel-other-side receiving part
201: Barrel-one-side receiving part
20A: Barrel projection
20B: Urging base part
20C: Penetrating part
20D: Barrel step part
21: Partition part
22: Plunger receiving part
220: Plunger-one-side receiving part
221: Plunger-other-side receiving part
22A: Plunger-receiving projection
22B: Plunger receiving recess
22B: Plunger receiving recess
23: Trigger part
230: Trigger mounting part
231: Trigger mounting base part
232: Trigger tubular part
233: Lock step part
234: Projection,
23A: Trigger body part
23B: Top plate part
23C: Outer skirt part
23D: Release groove
23E: Interlocking groove
23F: Inner skirt part
23G: Flat plate part
23a: Trigger lock operation part
23b: Tubular part
23c: Protrusion
23d: Rotation groove
3: Plunger urging part
4: Barrel urging part
5: Syringe
50: Barrel
500: Barrel body part
501: Barrel tubular part
502: Outer flange
503: Injection needle
50A: Cover

## Claims

1. An injection device comprising:
a barrel having a distal end with an injection needle;
a syringe comprising a plunger that is inserted into the barrel so as to be reciprocable between a proximal end side of the barrel as a starting point and a distal end side of the barrel with the injection needle as a terminal end point;
a housing having a tubular shape for receiving the syringe, the housing comprising a barrel receiving part on one side in a center axis direction so as to receive the barrel in a movable manner between a position at which the injection needle is received in the housing, and a position at which the barrel which has moved a certain distance from the position with the injection needle received toward one side to have the injection needle protruding from the one side of the housing, and a plunger receiving part on another side of the barrel receiving part to receive the plunger;
a barrel urging part arranged in the barrel receiving part and configured to urge the barrel from the one side to the other side of the housing;
an engaging member arranged in the plunger receiving part and configured to engage with the plunger;
a plunger urging part configured to engage with the engaging member and urge the plunger from the starting point toward the terminal end point;
an engagement releasing mechanism configured to release engagement between the plunger and the engaging member by rotating the plunger and the engaging member around a center axis with respect to each other in a state in which the barrel is received in the barrel receiving part with the injection needle protruding from the one side of the housing, and the plunger is positioned at the terminal end point due to an urging force of the plunger urging part; and
a rotation restricting mechanism configured to restrict a rotation speed of relative rotation between the plunger and the engaging member in the engagement releasing mechanism.

2. The injection device according to claim 1, wherein the engagement releasing mechanism is configured to release the engagement between the plunger and the engaging member by allowing the engaging member to rotate around the center axis with respect to the plunger, and the rotation restricting mechanism is configured to restrict the rotation speed of the engaging member.

3. The injection device according to claim 2, wherein the engaging member comprises an engaging rotation part that is configured to engage with the plunger and rotate around the center axis with respect to the plunger to release engagement with the plunger;
the engagement releasing mechanism is configured to release the engagement between the plunger and the engaging member by allowing the engaging rotation part to rotate with respect to the plunger to release the engagement between the plunger and the engaging member; and
the rotating restricting mechanism comprises an engaging non-rotation part that does not rotate around the center axis with respect to the engaging rotation part, and a rotation restricting part that is arranged between the engaging rotation part and the engaging non-rotation part and is configured to restrict the rotation speed of the engaging rotation part with respect to the engaging non-rotation part to restrict the rotation speed of the engaging rotation part with respect to the engaging non-rotation part.

4. The injection device according to claim 3, wherein the rotation restricting part is an elastic body arranged in an elastically deformed state between the engaging rotation part and the engaging non-rotation part.

5. The injection device according to claim 1, wherein the engagement releasing mechanism is configured to allow the plunger to rotate around the center axis with respect to the engaging member to release the engagement between the plunger and the engaging member; and
the rotation restricting mechanism is configured to restrict the rotation speed of the plunger.

6. The injection device according to claim 5, wherein the plunger comprises: a plunger rotation part that is configured to engage with the engaging member, and rotate around the center axis with respect to the engaging member to release the engagement;
a plunger non-rotation part that does not rotate around the center axis with respect to the plunger rotation part; and
a rotation restricting part that is arranged between the plunger rotation part and the plunger non-rotation part, and is configured to restrict the rotation speed of the plunger rotation part with respect to the plunger non-rotation part, wherein
the engagement releasing mechanism is configured to release the engagement between the engaging member and the plunger rotation part by allowing the plunger rotation part to rotate with respect to the engaging member to release the engagement between the plunger and the engaging member, and
the rotation restricting mechanism comprises the plunger non-rotation part and the rotation restricting part, and is configured to restrict the rotation speed of the plunger rotation part with respect to the plunger non-rotation part.

7. The injection device according to claim 6, wherein the rotation restricting part is an elastic body arranged in an elastically deformed state between the plunger rotation part and the plunger non-rotation part.
